(19) Europäisches Patentamt
European Patent Office
Office européen des brevets

(11) EP 0 834 490 A1

(12) **EUROPEAN PATENT APPLICATION**
published in accordance with Art. 158(3) EPC

(43) Date of publication:
**08.04.1998 Bulletin 1998/15**

(21) Application number: **96917682.5**

(22) Date of filing: **14.06.1996**

(51) Int. Cl.$^6$: **C07C 13/28**, C07C 15/50, C07C 15/52, C07C 22/04, C07C 25/24, C07C 43/192, C07C 43/225, C07C 43/188, C07C 43/215, C07C 69/757, C07C 69/75

(86) International application number:
**PCT/JP96/01626**

(87) International publication number:
**WO 97/00233 (03.01.1997 Gazette 1997/02)**

(84) Designated Contracting States:
**DE FR GB**

(30) Priority: **15.06.1995 JP 174487/95**

(71) Applicant: **CHISSO CORPORATION**
**Osaka-shi Osaka 530 (JP)**

(72) Inventors:
- **MIYAZAWA, Kazutoshi**
  **Chiba-ken 290-01 (JP)**
- **SATO, Hideo**
  **Yokohama-shi, Kanagawa-ken 236 (JP)**
- **KOBAYASHI, Katsuhiko**
  **Yokohama-shi, Kanagawa-ken 236 (JP)**
- **HACHIYA, Norihisa**
  **Ichihara-shi, Chiba-ken 299-01 (JP)**
- **NAKAGAWA, Etsuo**
  **Chiba-ken 290 (JP)**
- **MATSUI, Shuichi**
  **Ichihara-shi, Chiba-ken 290 (JP)**

(74) Representative:
**Ziebig, Marlene, Dr. Dipl.-Chem. et al**
**Patentanwälte**
**Gulde Hengelhaupt Ziebig**
**Lützowplatz 11-13**
**10785 Berlin (DE)**

# (54) COMPOUNDS HAVING ALKYL-SUBSTITUTED ALKENYLENE MOIETY AND LIQUID CRYSTAL COMPOSITION

(57) A compound of the formula (1), and a liquid crystal composition comprising the same:

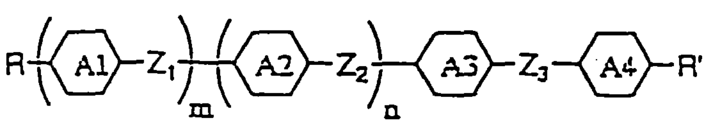
(1)

wherein R and R ' are alkyl groups having 1 to 10 carbon atoms, cyano groups, halogen atoms; rings $A_1$, $A_2$, $A_3$ and $A_4$ are benzene, cycloalkane having 3 to 8 carbon atoms; at least one of $Z_1$, $Z_2$ and $Z_3$ is an alkenylene group of the formula Q:

EP 0 834 490 A1

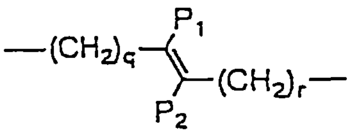

Q

wherein $P_1$ and $P_2$ are hydrogen atoms or alkyl groups having 1 to 10 carbon atoms, provided that at least one of $P_1$ and $P_2$ is alkyl group having 1 to 10 carbon atoms, q and r are 0 to 2 and the sum of q + r is 2 or less; and the other of $Z_1$, $Z_2$ and $Z_3$ are $-CH_2CH_2-$, m and n are 0 or 1. The liquid crystal compound of the invention has broader temperature range of a liquid crystal phase, excellent compatibility with other liquid crystal compounds, high stability and large elastic coefficient ratio $K_{33}/K_{11}$.

## Description

Prior art

This invention relates to a novel compound having various characteristics suitable for electrooptical display material, a liquid crystal composition using the compound and having the suitable various characteristics and a liquid crystal display element comprising the liquid crystal composition.

Background of the invention

Liquid crystal display elements are used in watches, electronic calculator, various kinds of measuring apparatuses, automotive panels, word processors, electronic notebooks, printers, computers, televisions, etc. Liquid crystal display elements utilize optical anisotropy and dielectric anisotropy of a liquid crystal compound. Display driving systems now often used include twisted nematic system (TN), super twisted nematic system (STN) and thin film transistor system (TFT).

Although various characteristics are required for liquid crystal materials according to display and driving systems, the following characteristics are important in common for all the systems:

1) broad temperature range of a liquid crystal phase, and
2) good compatibility with other liquid crystal compounds.

Among them, the characteristics 1) include high upper limit of nematic phase temperature, and low melting temperature so that phase separation such as separation of crystal hardly occurs at a low temperature region.

In other words, a liquid crystal composition comprises several to more than 20 kinds of liquid crystal compounds to develop optimum characteristics required for individual display element. For this purpose, a liquid crystal compound is required to have good compatibility with other liquid crystal compounds, and recently in particular, to have good compatibility at a lower temperature to meet the requirement for use under various circumstances.

Although characteristics required for liquid crystal compounds used in various liquid crystal display elements vary depending on the use, all the liquid crystal compounds are required to be stable against ambient factors such as moisture, air, heat, light, etc.

Namely, the most important characteristics required for liquid crystal compounds are the following three: 1) broad temperature range of liquid crystal phase, 2) good compatibility with other liquid crystal compounds, and 3) high stability.

In addition to the above, a composition used for STN drive is required to have steep threshold property for realizing high quality image. The steep threshold property is a function of an elastic coefficient ratio: $K_{33}/K_{11}$. It is known that the greater the ratio of the liquid crystal compound used in the liquid crystal composition, the steeper the threshold property of the composition (F. Leenhouts et al., Proceedings of the Japan Display, 388(1986)) .

A liquid crystal composition for active matrix liquid crystal display which comprises an integrated nonlinear element for switching individual image points, in particular, a liquid crystal composition designed for TFT, must have very high specific resistance (high voltage holding ratio), good UV stability, high compatibility with other liquid crystal compound at a low temperature in addition to large positive dielectric anisotropy.

Active matrix liquid crystal display is suitable for, in particular, advanced information displays for television or computer, or advanced information displays used in automobiles or aircrafts. However, if a liquid crystal compound or a liquid crystal composition which does not have very high specific resistance (high voltage holding ratio), and good UV stability, is used, electrical resistance in a liquid crystal panel is reduced which leads to reduction of contrast to thereby cause "after image extinction". High electrical resistance of a liquid crystal composition is very important factor which governs a use-life in case of low voltage drive. Accordingly, very high specific resistance (high voltage holding ratio), and good UV stability are very important characteristics required for a liquid crystal compound used.

Thus, there is a need for a liquid crystal compound having:

1) broader temperature range of a liquid crystal phase,
2) better compatibility with other liquid crystal compounds, and
3) higher stability

than liquid crystal compounds known in the art.

Further, there is a need for a liquid crystal compound used in an STN liquid crystal composition, which has 1) higher elastic coefficient ratio: $K_{33}/K_{11}$ and a liquid crystal compound used in a TFT liquid crystal composition, which has 1) higher specific resistance (higher voltage holding ratio) and better UV stability.

Disclosure of the invention

Thus, an object of the invention is to provide a liquid crystal compound having broader temperature range of liquid crystal phase, better compatibility with other liquid crystal compounds, and higher stability than liquid crystal compounds known in the art. Another object of the invention is to provide a liquid crystal compound used in an STN liquid crystal composition, which has higher elastic coefficient ratio: $K_{33}/K_{11}$ and a liquid crystal compound used in a TFT liquid crystal composition, which has higher specific resistance (higher voltage holding ratio) and better UV stability.

The inventors of the present invention have studied to attain the above mentioned objects and found a compound which has a novel structure and improved characteristics as compared with known liquid crystal compounds, based on which they have completed the present invention. That is, the above objects can be attained by a compound obtainable by introducing alkyl-substituted alkenylene moiety as a central linking group.

The present invention provides a compound of the following formula (1):

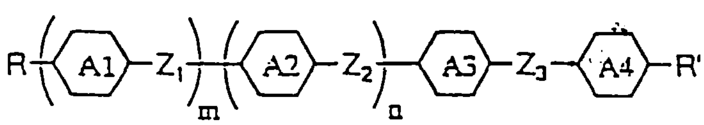

(1)

wherein R and R 'represent independently alkyl groups having 1 to 10 carbon atoms, cyano groups, halogen atoms, halogenated alkyl groups having 1 to 5 carbon atoms, or said alkyl groups or halogenated alkyl groups wherein, at least one $-CH_2-$ is replaced by -O-, -S-, -CH=CH- or -C≡C-;
rings $A_1$, $A_2$, $A_3$ and $A_4$ represent independently benzene rings, cycloalkane rings having 3 to 8 carbon atoms, cycloalkene rings having 3 to 8 carbon atoms, bicycloalkane rings having 5 to 10 carbon atoms, benzene rings wherein at least one -CH= in the rings is replaced by -N=, -CF=, -CCl= or -CBr=, cycloalkane rings wherein at least one $-CH_2-$ in the rings is replaced by -O- or -S-, cycloalkene rings wherein at least one $-CH_2-$ in the rings is replaced by -O- or -S- and/or at least one - CH= in the rings is replaced by -N=, or bicycloalkane rings wherein at least one $-CH_2-$ in the rings is replaced by -O- or -S- and/or at least one -CH= in the rings is replaced by -N=;

at least one of $Z_1$, $Z_2$ and $Z_3$ represents an alkenylene group of the following formula Q:

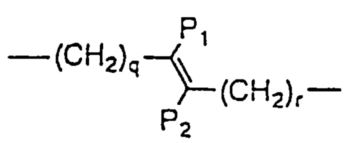

Q

wherein $P_1$ and $P_2$ represent independently hydrogen atoms or alkyl groups having 1 to 10 carbon atoms, provided that at least one of $P_1$ and $P_2$ represent alkyl groups having 1 to 10 carbon atoms, q and r represent independently 0, 1 or 2 and the sum of q + r is 2 or less; and the other of $Z_1$, $Z_2$ and $Z_3$ represent independently a covalent bond, $-CH_2CH_2-$, - CH=CH-, -C≡C-, -COO-, -OCO-, $-CH_2O-$, $-OCH_2-$, $-CF_2O-$, $-OCF_2-$, $-(CH_2)_4-$, $-CH=CH-(CH_2)_2-$, $-CH_2CH=CHCH_2-$ or $-(CH_2)_2-CH=CH-$;
m and n represent independently 0 or 1, provided that if m is 0, at least one of $Z_2$ and $Z_3$ is Q and if both m and n are 0, $Z_3$ is Q.

Preferred compounds of the present invention are those of the formula wherein one of $P_1$ and $P_2$ is methyl group and the other is hydrogen atom. More preferred compounds are those of the formula wherein both q and r are 0.

The present invention also provides a liquid crystal composition which comprises at least one compound of the formula (1).

The present invention further provides a liquid crystal composition which comprises at least one compound of the formula (1) as a first component and at least one compound selected from the group consisting of the compounds of following formulas (2), (3) and (4) as a second component.

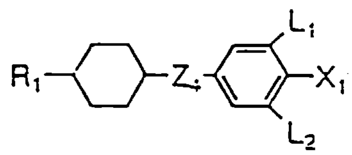

(2)

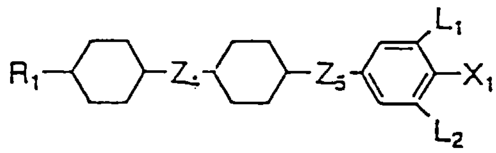

(3)

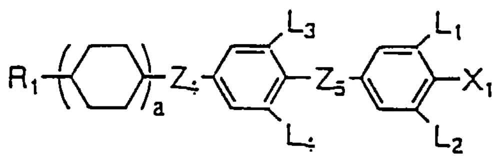

(4)

wherein $R_1$ represents an alkyl group having 1 to 10 carbon atoms, $X_1$ represents F, Cl, $OCF_3$, $OCF_2H$, $CF_3$, $CF_2H$ or $CFH_2$, $L_1$, $L_2$, $L_3$ and $L_4$ represent independently H or F, $Z_4$ and $Z_3$ represent independently - $(CH_2)_2$-, -CH=CH- or covalent bonds, and a is 1 or 2.

The present invention further provides a liquid crystal composition which comprises at least one compound of the formula (1) as a first component and at least one compound selected from the group consisting of the compounds of following formulas (5), (6), (7), (8) and (9) as a second component.

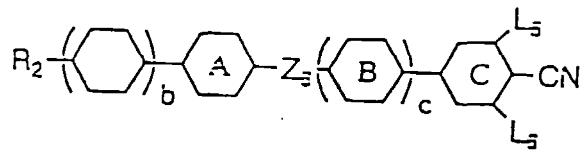

(5)

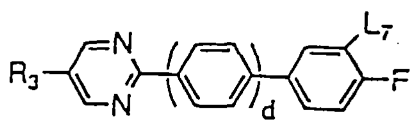

(6)

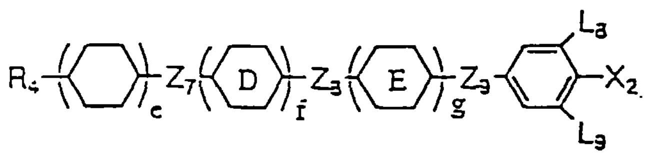

(7)

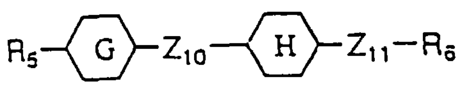

(8)

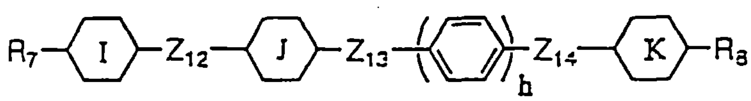

(9)

In the formula (5), $R_2$ is F or an alkyl group having 1 to 10 carbon atoms or an alkenyl group having 2 to 10 carbon atoms. Any methylene group ($-CH_2-$) in said alkyl or alkenyl group may be replaced by oxygen atom (-O-), provided that two or more successive methylene groups are not replaced by oxygen atoms. Ring A represents trans-1,4-cyclohexylene, 1,4-phenylene or 1,3-dioxane-2,5-diyl group, ring B represents trans-1,4-cyclohexylene, 1,4-phenylene or pyrimidine-2,5-diyl group, ring C represents trans-1,4-cyclohexylene, or 1,4-phenylene group, $Z_6$ represents $-(CH_2)_2-$, -COO- or a covalent bond, $L_5$ and $L_6$ represent independently H or F, and b and c represent independently 0 or 1.
In the formula (6), $R_3$ represents an alkyl group having 1 to 10 carbon atoms, $L_7$ represents H or F, and d is 0 or 1.
In the formula (7), $R_4$ represents an alkyl group having 1 to 10 carbon atoms, rings D and E represent independently trans-1,4-cyclohexylene or 1,4-phenyelne group, $Z_7$ and $Z_8$ represent independently -COO- or covalent bonds, $Z_9$ represents -COO- or -C≡C-, $L_8$ and $L_9$ represent independently H or F, $X_2$ represents F, $OCF_3$, $OCF_2H$, $CF_3$, $CF_2H$ or $CFH_2$, provided that if $X_2$ is $OCF_3$, $OCF_2H$, $CF_3$, $CF_2H$ or $CFH_2$, both $L_8$ and $L_9$ are H. e, f and g represent independently 0 or 1.
In the formula (8), $R_5$ and $R_6$ represent independently alkyl groups having 1 to 10 carbon atoms or alkenyl groups having 2 to 10 carbon atoms. Any methylene group ($-CH_2-$) in said alkyl or alkenyl group may be replaced by oxygen atom (-O-), provided that two or more successive methylene groups are not replaced by oxygen atoms. Ring G represents trans-1,4-cyclohexylene, 1,4-phenylene or pyrimidine-2,5-diyl group, ring H represents trans-1,4-cyclohexylene or 1,4-phenylene group, $Z_{10}$ represents -C≡C-, -COO-, $-(CH_2)_2-$, -CH=CH-C≡C- or a covalent bond, $Z_{11}$ represents -COO- or a covalent bond.

In the formula (9), $R_7$ and $R_8$ represent independently alkyl groups having 1 to 10 carbon atoms or alkenyl groups having 2 to 10 carbon atoms. Any methylene group ($-CH_2-$) in said alkyl or alkenyl group may be replaced by oxygen atom (-O-), provided that two or more successive methylene groups are not replaced by oxygen atoms. Ring I represents trans-1,4-cyclohexylene, 1,4-phenylene or pyrimidine-2,5-diyl group, ring J represents trans-1,4-cyclohexylene group, 1,4-phenylene group wherein one or more hydrogen atoms on the ring may be replaced by F, or pyrimidine-2,5-diyl group wherein one or more hydrogen atoms on the ring may be replaced by F, ring K represents trans-1,4-cyclohexylene or 1,4-phenylene group, $Z_{12}$ and $Z_{14}$ represent independently -COO-, $-(CH_2)_2-$or covalent bonds, $Z_{13}$ represents -CH=CH-, -C ≡ C-, -COO- or a covalent bond, and h is 0 or 1.

The present invention further provides a liquid crystal composition which comprises at least one compound of the formula (1) as a first component, at least one compound selected from the group consisting of the compounds of the formulas (2), (3) and (4) as a second component and at least one compound selected from the group consisting of the compounds of the formulas (5), (6), (7), (8) and (9) as aother of a second component.

The present invention further provides a liquid crystal display element comprising the liquid crystal composition mentioned above.

Best mode for carrying out the invention

The compounds of the formula (1) of the present invention has broad liquid crystal phase temperature range, good compatibility with other liquid crystal compounds and very high chemical stability. More specifically, they have high voltage holding ratio, good UV stability and high compatibility with other liquid crystal compounds at low temperature. Further, the liquid crystal composition comprising the liquid crystal compound of the formula (1) has steep threshold voltage characteristics, highly accurate and fine display quality, and long life and is able to drive at low temperature.

As seen from the comparative examples described later, the excellent characteristics of the present invention, in particular, broad liquid crystal phase temperature range are due to the novel partial structure, i.e.,the alkyl-substituted alkenylene moiety.

JP-B-3-22855 and JP-A-63-502756 disclose a compound having no branched structure in the central linking site, and a saturated alkylene compound having branched structure, respectively. However, there is a need for a liquid crystal compound having much broader liquid crystal phase temperature range, in particular, a liquid crystal compound having much higher upper temperature limit for nematic phase.

All of the compounds of the present invention show excellent characteristics. However, those compounds of the formula (1) wherein R, R', rings $A_1$, $A_2$, $A_3$ and $A_4$, $Z_1$, $Z_2$, $Z_3$, m, n, $P_1$, $P_2$, q and r are suitably selected are used to prepare a liquid crystal composition having intended characteristics.

Namely, it is appropriate to use three or four rings compounds, if a liquid crystal composition having higher liquid crystal phase temperature range is desired, while it is appropriate to use two or three rings compounds for other liquid crystal compositions.

If a particularly large dielectric anisotropy is required, a compound having positive dielectric anisotropy (P-type compound) as used in conventional compositions is used. Such P-type compound is selected from those of the formula (1) wherein R' is halogen atom, cyano or halogenated alkyl group. If a much larger dielectric anisotropy is required, it is appropriate to use a compound of the formula (1) wherein a halogen atom is introduced into the ring so that electric dipole is in the same direction.

A compound having negative dielectric anisotropy (N-type compound) is provided by introducing a group having not large electric dipole moment such as alkyl or alkoxy group as R' in the formula (1).

Optical anisotropy can also voluntarily be adjusted by appropriately selecting R, R', rings $A_1$, $A_2$, $A_3$ and $A_4$, $Z_1$, $Z_2$, $Z_3$, m, n, $P_1$, $P_2$, q and r. Namely, if a large optical anisotropy is required, a compound having many 1,4-phenylene rings is used, while if a small optical anisotropy is required, a compound having many trans-1,4-cyclohexylene rings is used.

The term "alkyl group" herein used means a linear or branched alkyl group having 1 to 10 carbon atoms, in particular having 3 to 5 carbon atoms from the view point of low viscosity. Specifically, preferred are methyl, ethyl, propyl, butyl, pentyl, hexyl, heptyl, octyl, isopropyl, isobutyl, isoamyl, isohexyl, 2-methylbutyl, 2-methylpentyl, and 3-methylpentyl groups, including racemic body, S-form and R-form thereof.

The term "halogenated alkyl group" herein used means a group having 1 to 5 carbon atoms wherein at least one fluorine and/or chlorine atom is bonded to the carbon atom. Preferred are monofluoromethyl, difluoromethyl, trifluoromethyl, 2-fluoroethyl, 2,2-difluoroethyl, 2,2,2-trifluoroethyl, 1,1,2,2-tetrafluoloethyl, perfluoroethyl, 3-fluoropropyl, 3,3-difluoropropyl, 3,3,3-trifluoropropyl, 2,2,3,3,3-pentafluoropropyl, 1,1,2,3,3,3-hexafluoropropyl, perfluoropropyl, 4-fluorobutyl, perfluorobutyl and 5-fluoropentyl groups.

As long as Q meets the requirements mentioned above, all the compounds of the formula (1) show excellent characteristics. If, however, a compound having low viscosity is desired, $P_1$ and $P_2$ preferably are alkyl groups having 1 to 3 carbon atoms, more preferably one carbon atom. If much lower viscosity is required, it is suitable to use a compound of the formula wherein at least one of $P_1$ and $P_2$ is hydrogen atom. If broader nematic liquid crystal phase temperature

range is required, it is preferable to use a compound of the formula wherein two rings are bonded to the alkyl-substituted alkenylene moiety Q in the trans form.

Examples of $A_1$, $A_2$, $A_3$ and $A_4$ include benzene, cyclopropane, cyclobutane, cyclopentane, cyclohexane, pyrimidine, pyridine, pyrazine, pyridazine, bicyclopentane, bicyclooctane, dioxane rings and halogenated rings thereof, with benzene, cyclohexane and halogenated rings thereof being preferred.

The alkyl-substituted alkenylene moiety Q produces preferable characteristics as long as it meets the above requirements. Specific examples thereof include 1-methyethenylene, 1-ethylethenylene, 1-propylethenylene, 1-butylethenylene, 1-pentylethenylene, 1,2-dimethylethenylene, 1-methyl-2-ethylethenylene, 1-methyl-2-propylethenylene, 1-methyl-1-butenylene, 2-methyl-1-butenylene and 2-methyl-2-butenylene.

Among the compounds of the formula (1) of the present invention, preferred are the compounds of the formulas (1-a) to (1-d) wherein R, R', rings $A_1$, $A_2$, $A_3$ and $A_4$, $Z_1$, $Z_2$, $Z_3$, $P_1$, $P_2$, q and r are the same as defined above.

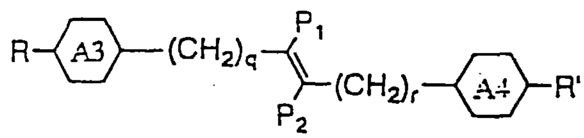

(1-a)

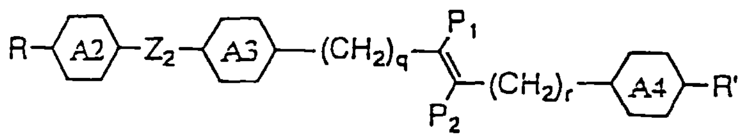

(1-b)

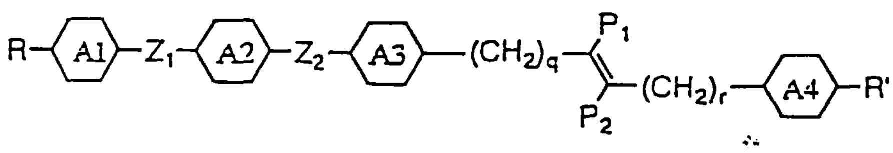

(1-c)

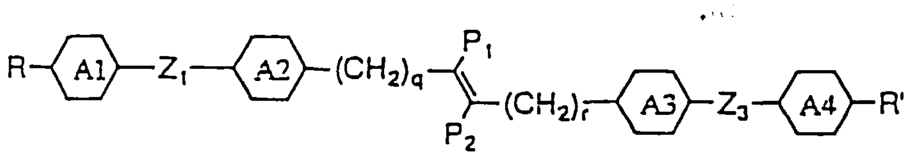

(1-d)

More preferred are the compounds of the formulas (1-a-1) to (1-d-4) wherein Ra and Rb represent alkyl, alkoxy or alkenyl groups having up to 10 carbon atoms.

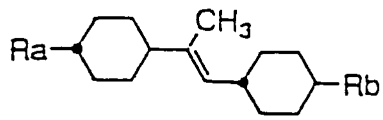

(1-a-1)

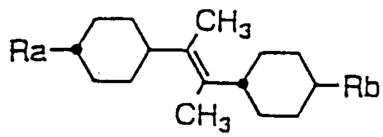

(1-a-2)

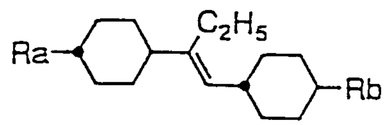

(1-a-3)

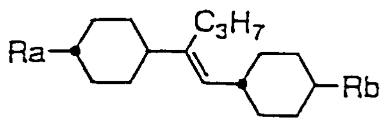

(1-a-4)

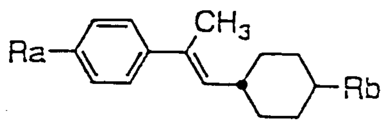

(1-a-5)

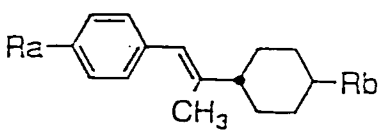

(1-a-6)

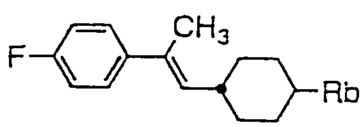

(1-a-7)

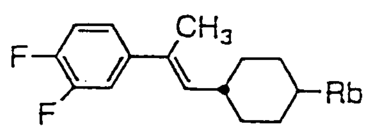

(1-a-8)

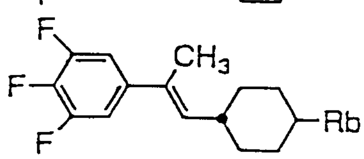

(1-a-9)

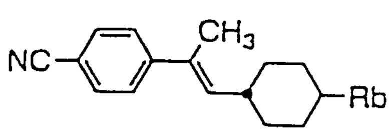

(1-a-10)

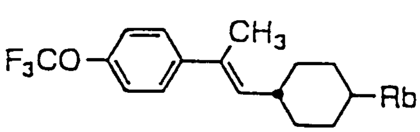

(1-a-11)

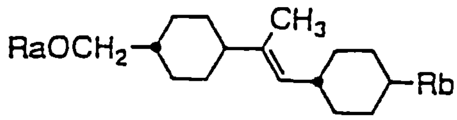
(1-a-12)

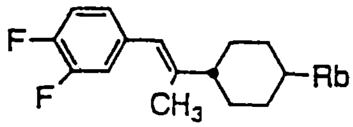
(1-a-13)

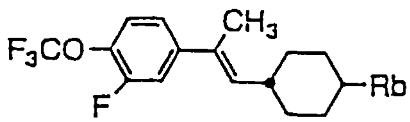
(1-a-14)

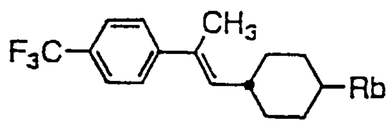
(1-a-15)

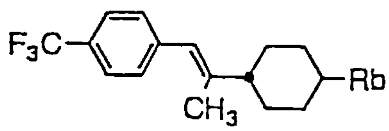
(1-a-16)

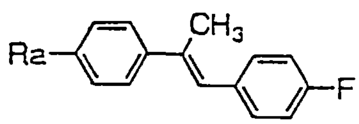
(1-a-17)

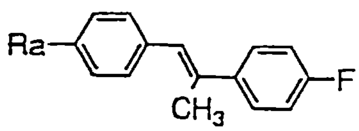
(1-a-18)

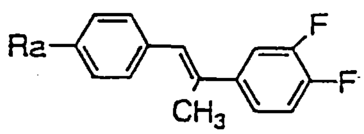
(1-a-19)

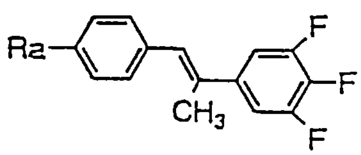
(1-a-20)

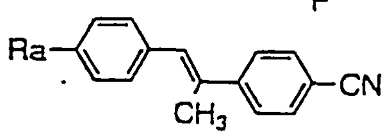
(1-a-21)

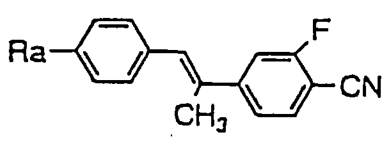
(1-a-22)

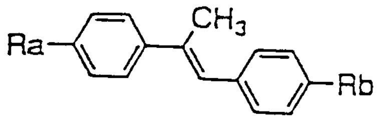
(1-a-23)

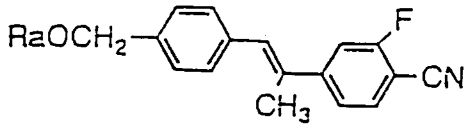
(1-a-24)

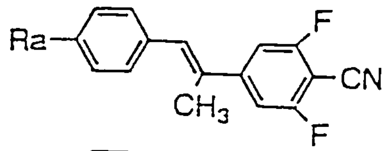
(1-a-25)

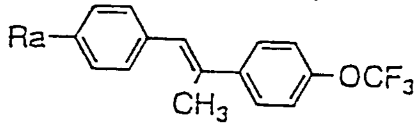
(1-a-26)

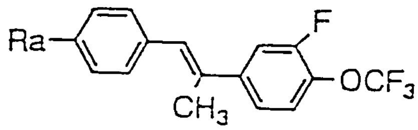
(1-a-27)

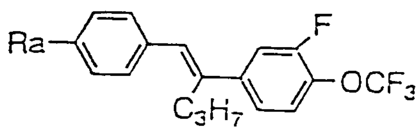
(1-a-28)

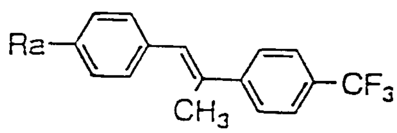
(1-a-29)

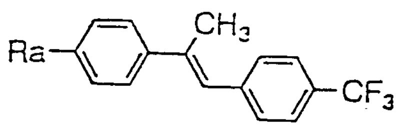
(1-a-30)

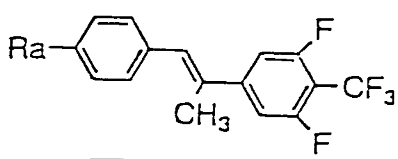
(1-a-31)

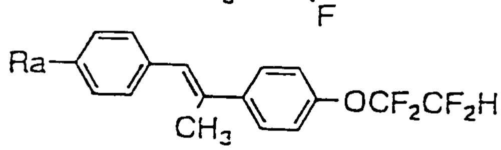
(1-a-32)

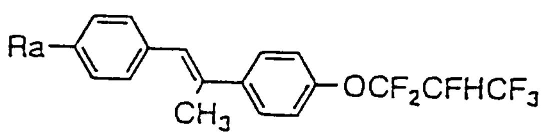
(1-a-33)

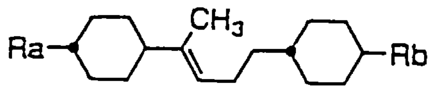

(1-a-34)

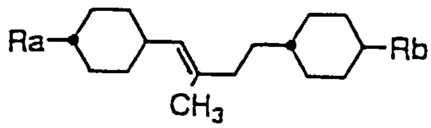

(1-a-35)

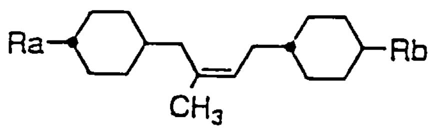

(1-a-36)

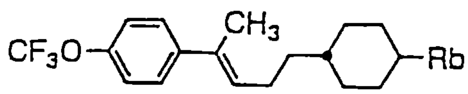

(1-a-37)

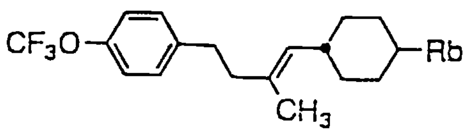

(1-a-38)

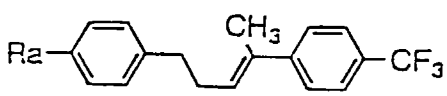

(1-a-39)

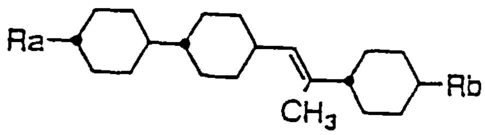

(1-b-1)

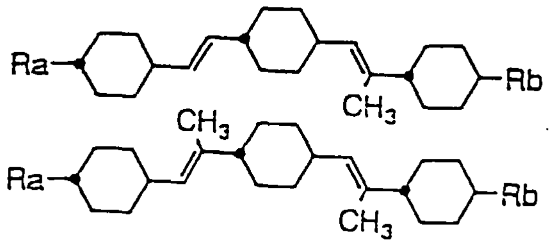

(1-b-2)

(1-b-3)

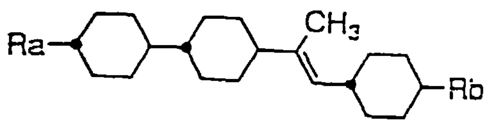

(1-b-4)

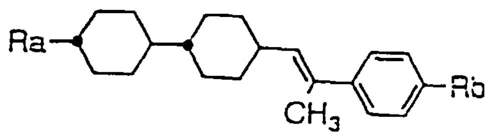

(1-b-5)

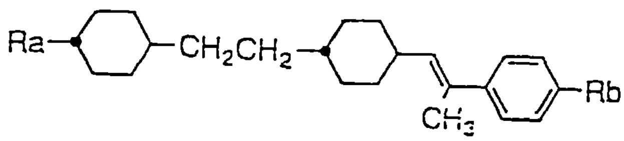

(1-b-6)

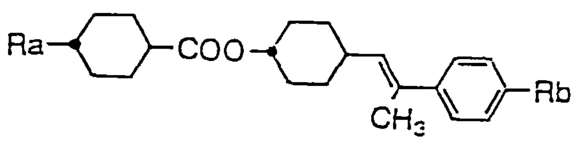

(1-b-7)

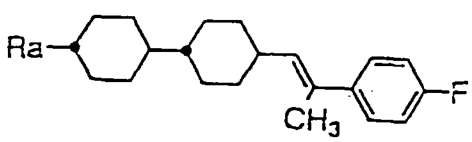

(1-b-8)

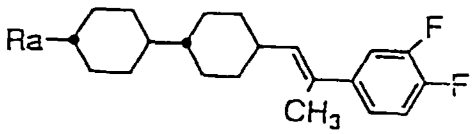

(1-b-9)

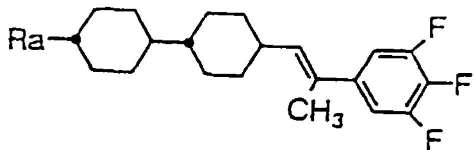

(1-b-10)

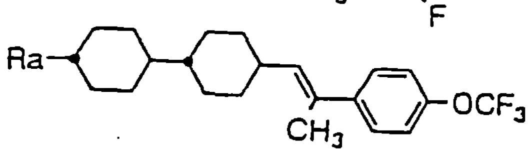

(1-b-11)

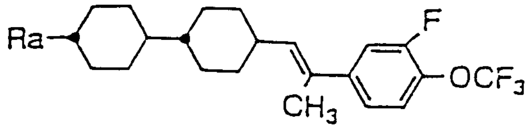

(1-b-12)

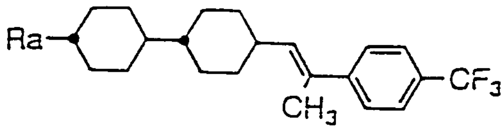

(1-b-13)

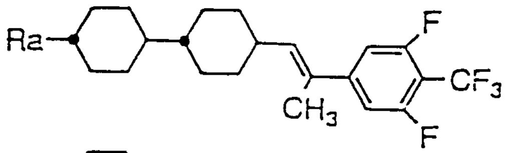

(1-b-14)

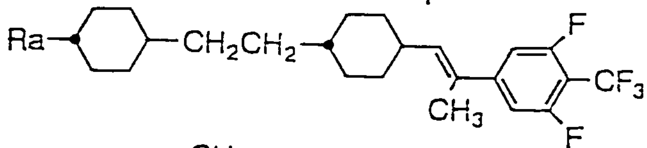

(1-b-15)

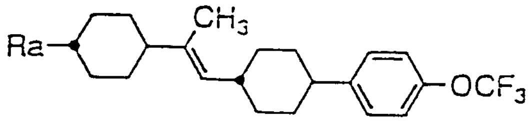

(1-b-16)

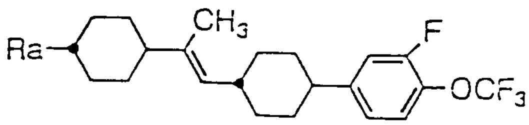

(1-b-17)

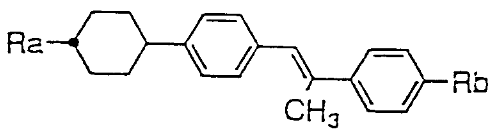

(1-b-18)

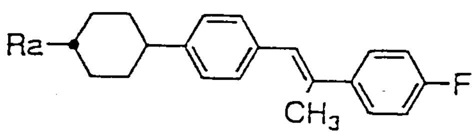

(1-b-19)

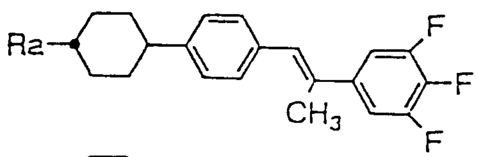

(1-b-20)

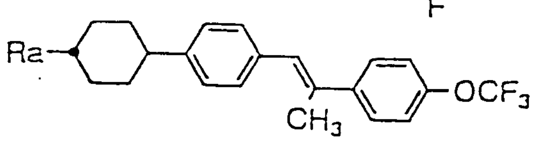

(1-b-21)

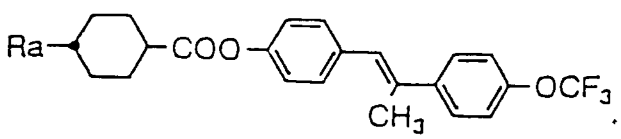

(1-b-22)

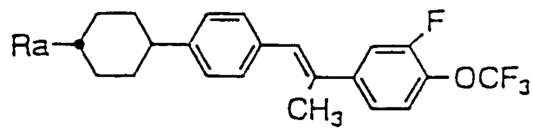 (1-b-23)

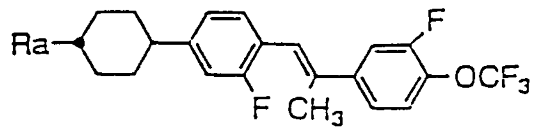 (1-b-24)

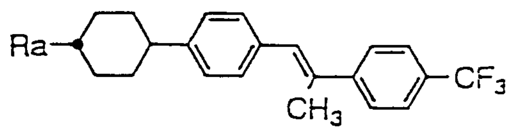 (1-b-25)

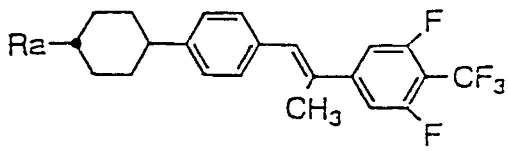 (1-b-26)

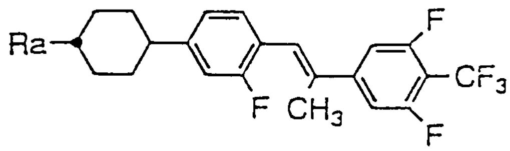 (1-b-27)

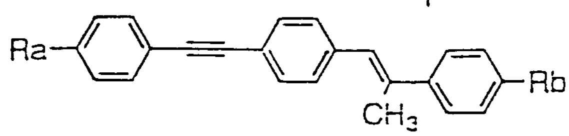 (1-b-28)

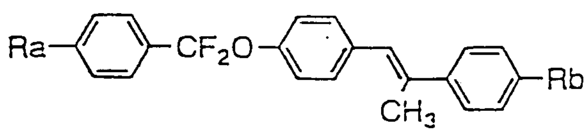 (1-b-29)

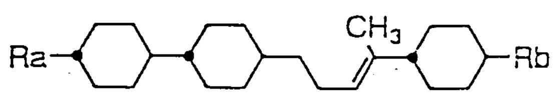 (1-b-30)

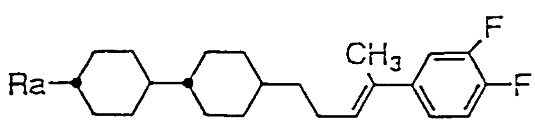 (1-b-31)

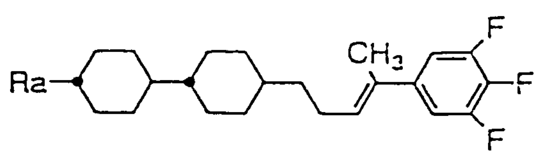 (1-b-32)

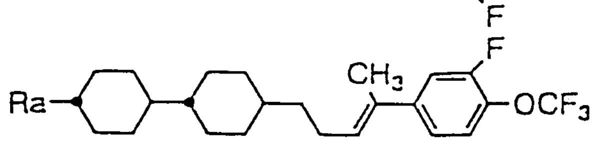 (1-b-33)

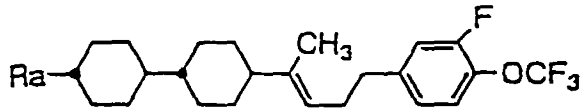

(1-b-34)

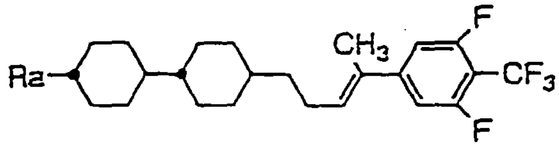

(1-b-35)

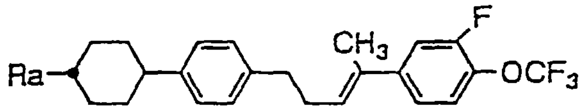

(1-b-36)

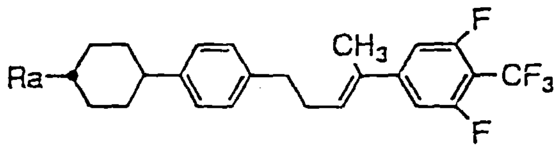

(1-b-37)

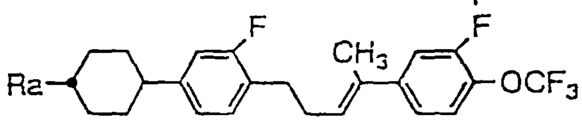

(1-b-38)

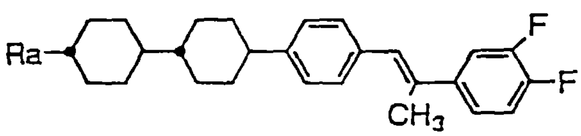

(1-c-1)

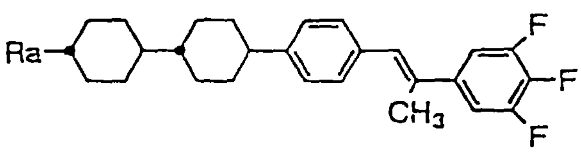

(1-c-2)

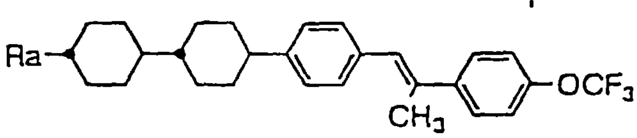

(1-c-3)

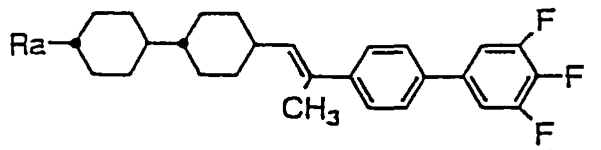 (1-d-1)

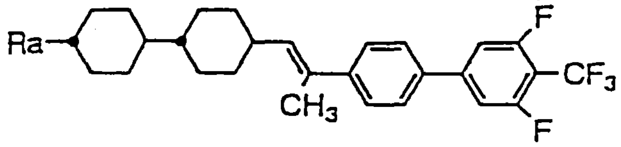 (1-d-2)

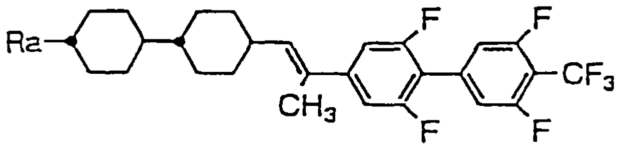 (1-d-3)

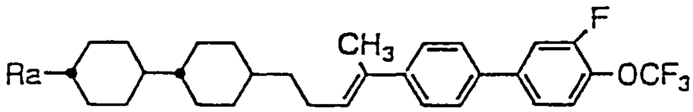 (1-d-4)

The liquid crystal composition of the present invention may contain at least one of the liquid crystal compounds of the formula (1) (hereinafter also referred to as "a first component") alone. Preferably, the liquid crystal composition of the present invention contains in addition to the first component, as a second component, at least one compound selected from the group consisting of the liquid crystal compounds of the formulas (2), (3) and (4) (hereinafter also referred to as "a second component A") and/or at least one compound selected from the group consisting of the liquid crystal compounds of the formulas (5), (6), (7), (8) and (9) (hereinafter also referred to as "a second component B"). Further, the composition may contain a known, compound, as a third component, to adjust threshold voltage, liquid crystal phase temperature range, optical anisotropy, dielectric anisotropy, and viscosity.

Among the second component A, preferred examples of the formulas (2), (3) and (4) are (2-1) to (2-15), (3-1) to (3-48) and (4-1) to (4-55), respectively.

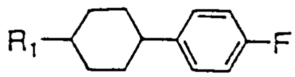 (2-1)

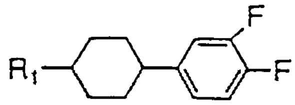 (2-2)

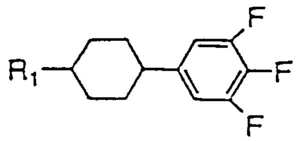 (2-3)

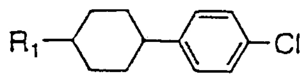 (2-4)

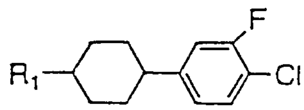 (2-5)

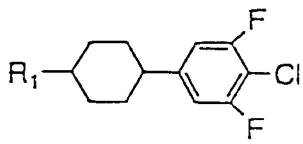 (2-6)

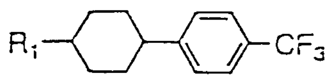 (2-7)

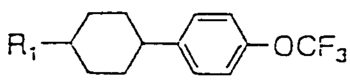 (2-8)

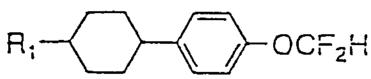 (2-9)

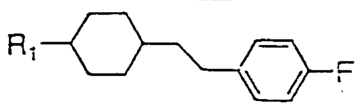 (2-10)

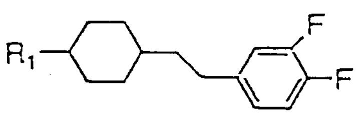 (2-11)

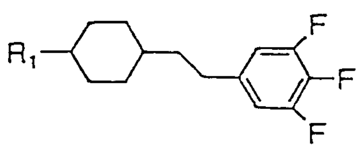 (2-12)

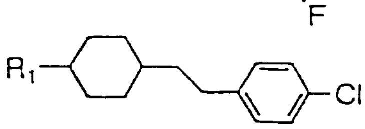 (2-13)

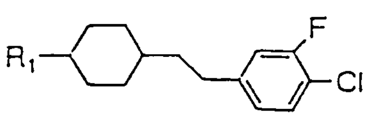 (2-14)

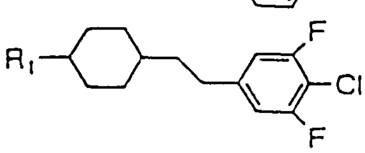 (2-15)

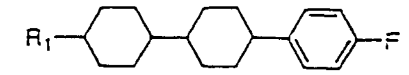
(3-1)

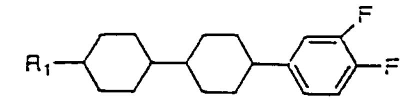
(3-2)

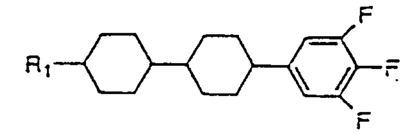
(3-3)

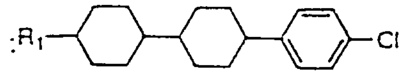
(3-4)

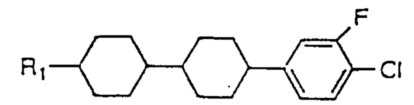
(3-5)

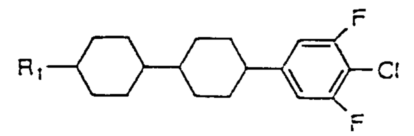
(3-6)

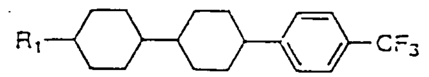
(3-7)

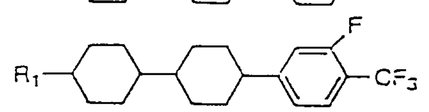
(3-8)

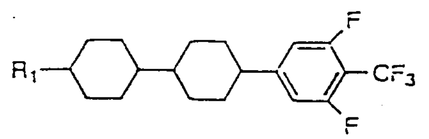
(3-9)

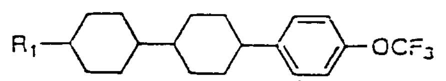
(3-10)

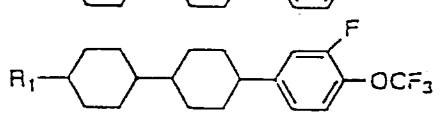
(3-11)

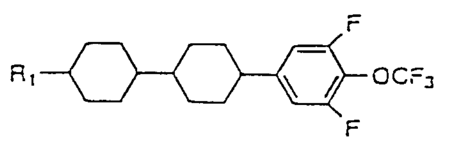
(3-12)

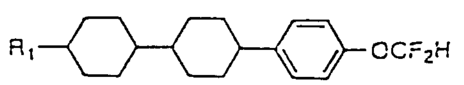
(3-13)

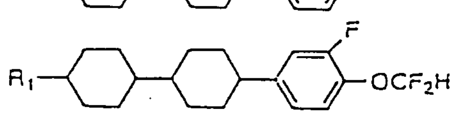
(3-14)

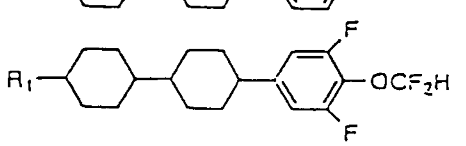
(3-15)

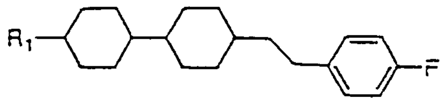
(3-16)

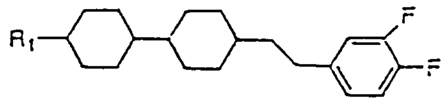
(3-17)

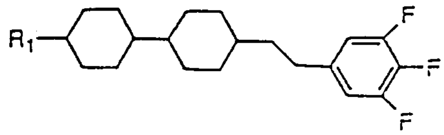
(3-18)

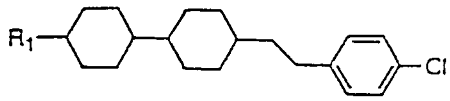
(3-19)

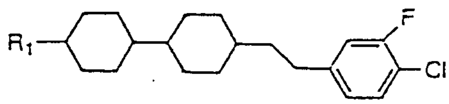
(3-20)

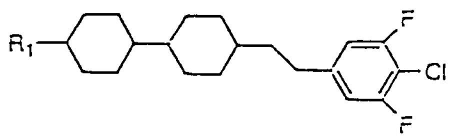
(3-21)

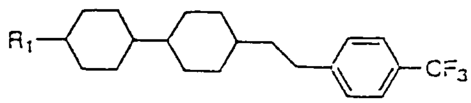
(3-22)

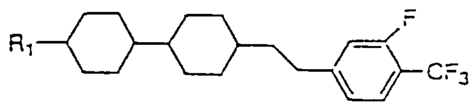
(3-23)

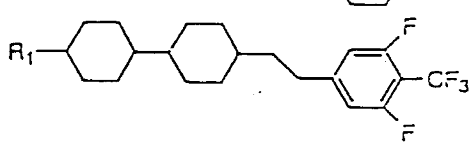
(3-24)

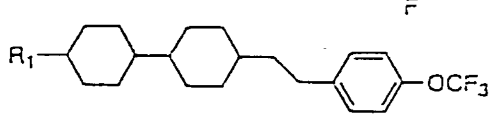
(3-25)

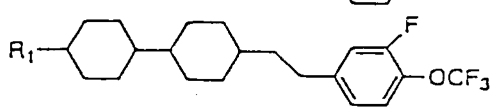
(3-26)

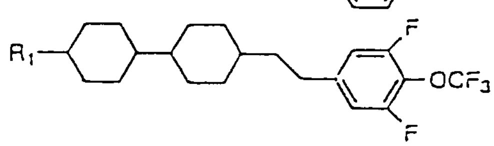
(3-27)

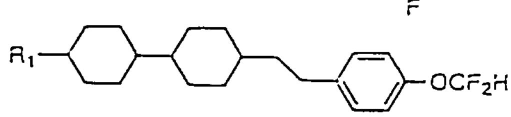
(3-28)

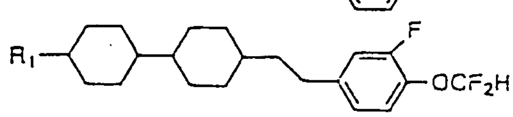
(3-29)

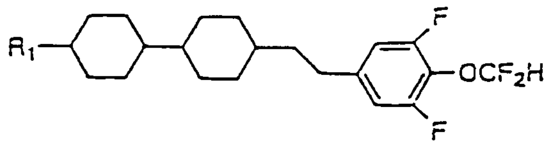
(3-30)

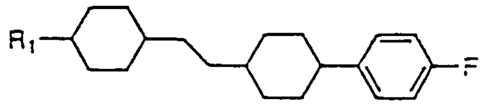
(3-31)

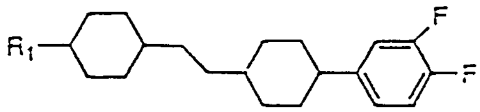
(3-32)

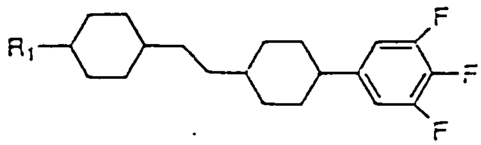
(3-33)

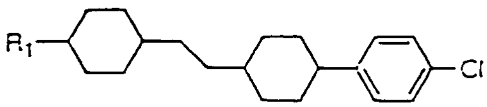
(3-34)

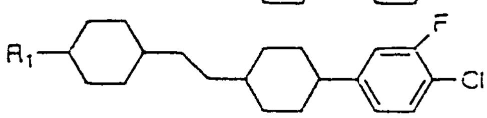
(3-35)

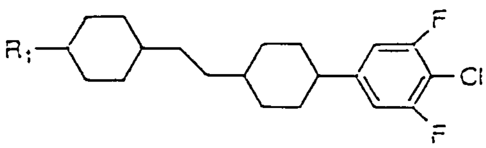
(3-36)

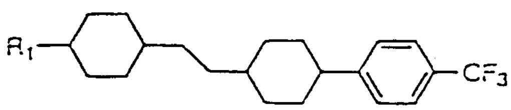
(3-37)

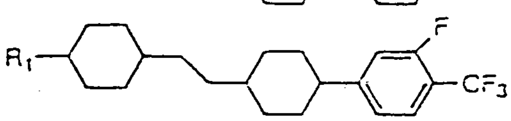
(3-38)

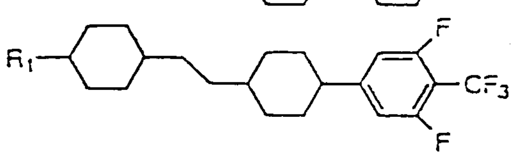
(3-39)

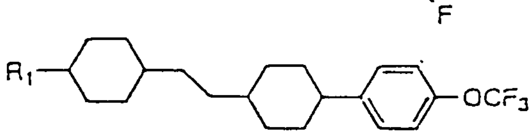
(3-40)

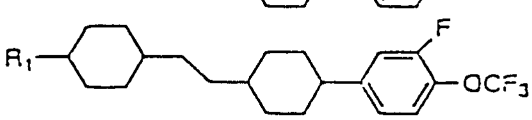
(3-41)

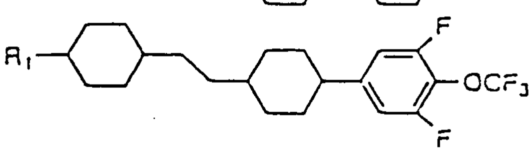
(3-42)

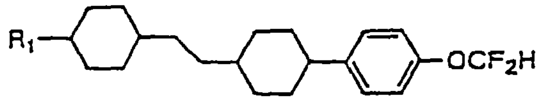

(3-43)

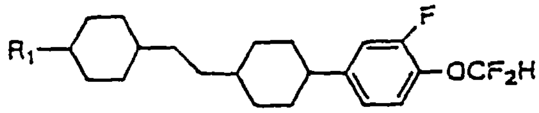

(3-44)

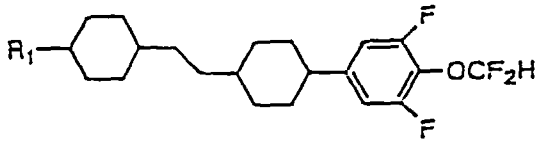

(3-45)

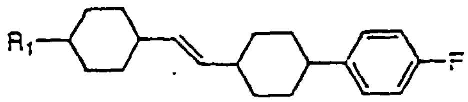

(3-46)

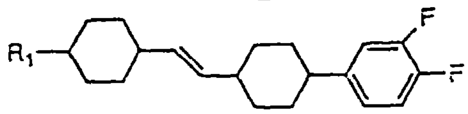

(3-47)

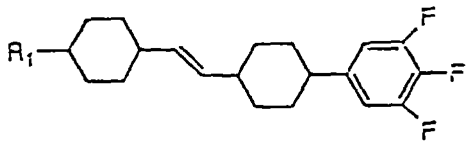

(3-48)

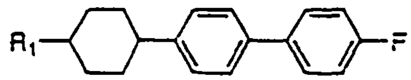

(4-1)

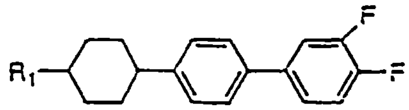

(4-2)

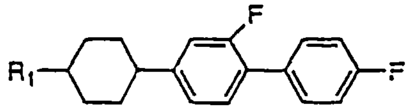

(4-3)

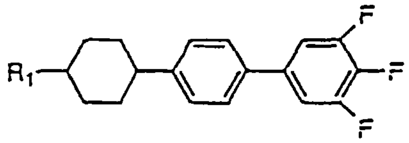

(4-4)

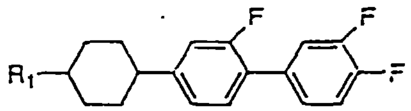

(4-5)

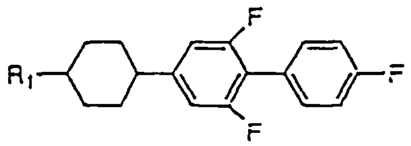

(4-6)

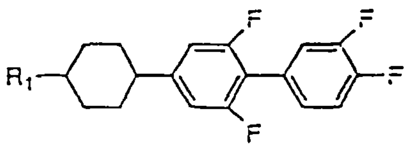

(4-7)

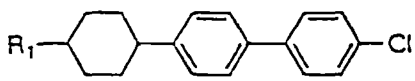

(4-8)

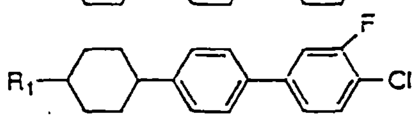

(4-9)

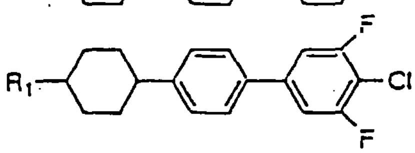

(4-10)

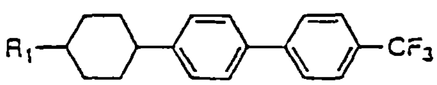

(4-11)

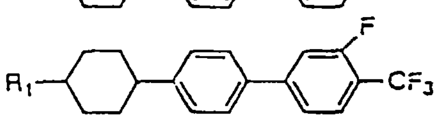

(4-12)

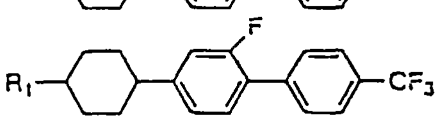

(4-13)

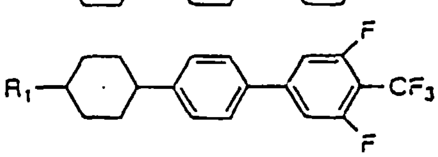

(4-14)

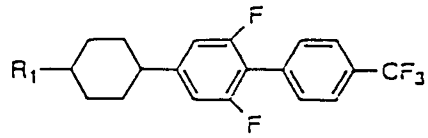

(4-15)

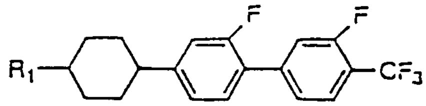

(4-16)

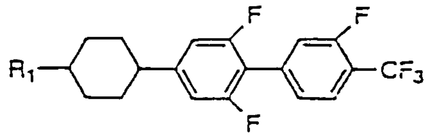

(4-17)

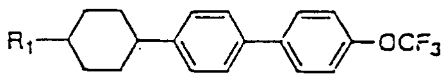

(4-18)

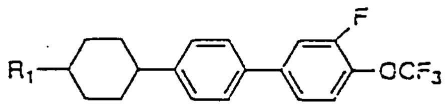

(4-19)

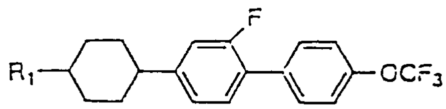

(4-20)

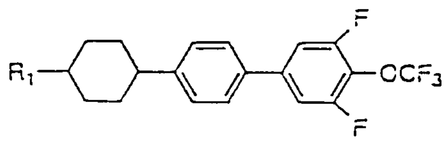

(4-21)

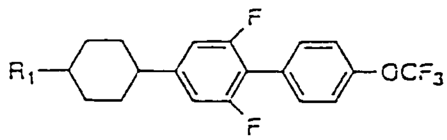

(4-22)

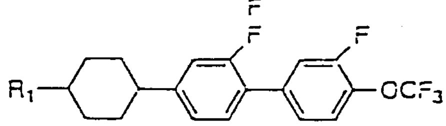

(4-23)

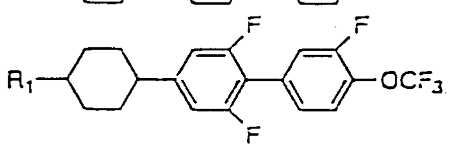

(4-24)

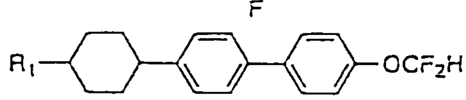

(4-25)

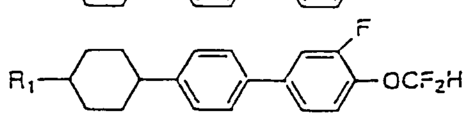

(4-26)

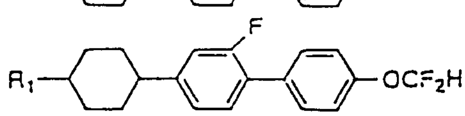

(4-27)

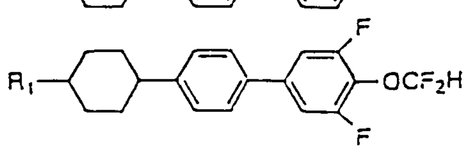

(4-28)

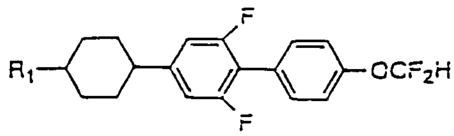

(4-29)

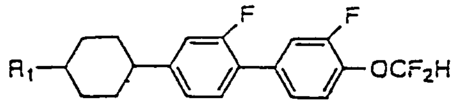

(4-30)

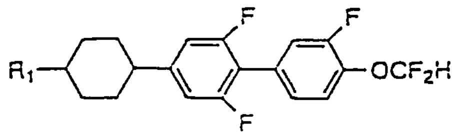

(4-31)

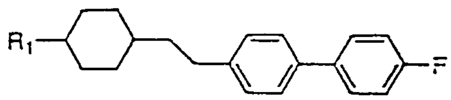

(4-32)

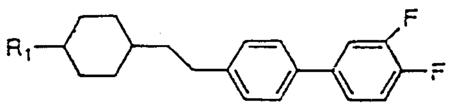

(4-33)

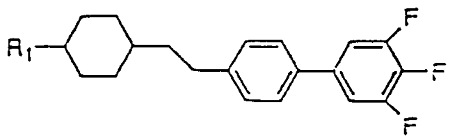

(4-34)

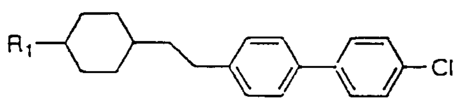

(4-35)

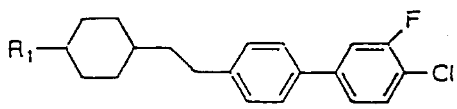

(4-36)

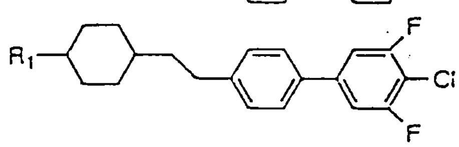

(4-37)

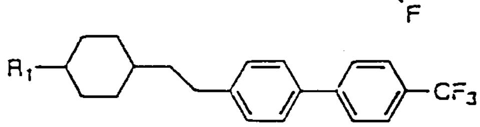

(4-38)

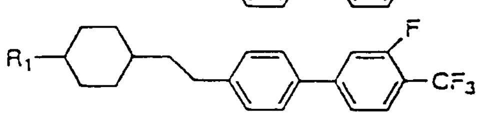

(4-39)

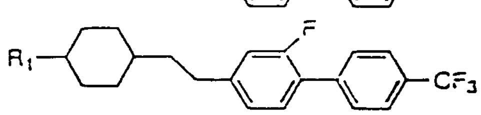

(4-40)

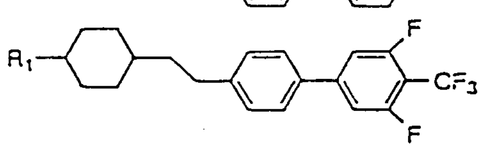

(4-41)

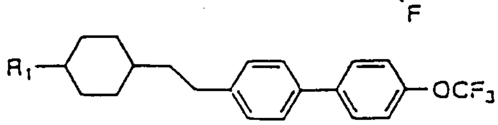

(4-42)

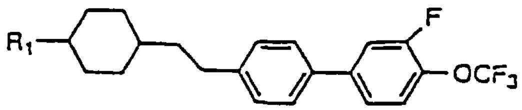

(4-43)

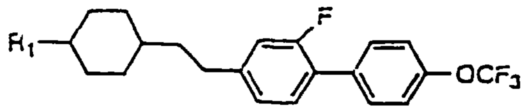

(4-44)

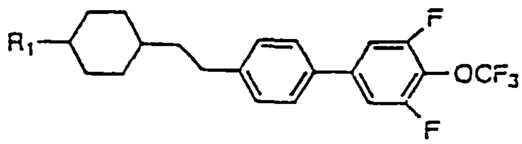

(4-45)

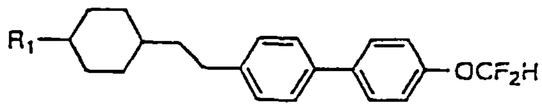

(4-46)

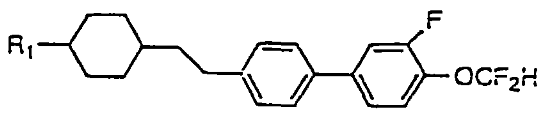

(4-47)

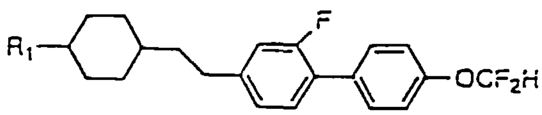

(4-48)

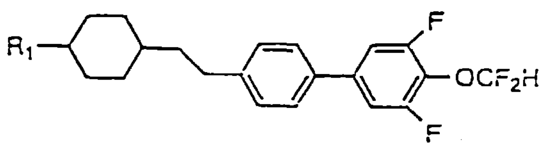

(4-49)

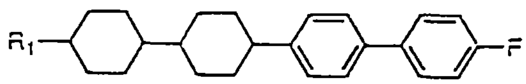

(4-50)

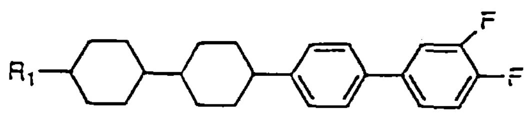

(4-51)

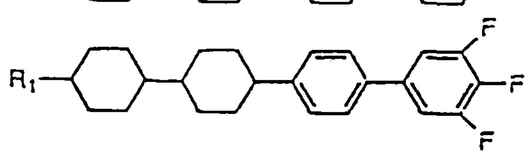

(4-52)

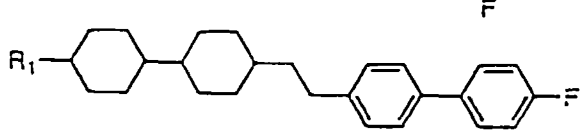

(4-53)

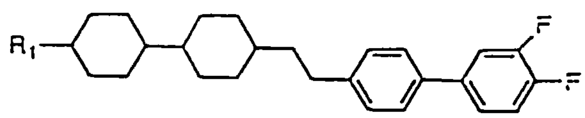

(4-54)

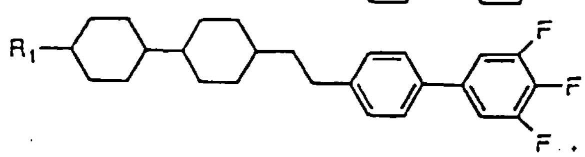

(4-55)

The compounds of the formulas (2) to (4) show positive dielectric anisotropy and have very good heat stability and chemical stability.

The amount of the compounds of the formulas (2) to (4) is suitably 1 to 99 % by weight, preferably 10 to 97 % by weight, and more preferably 40 to 95 % by weight based on the total weight of the liquid crystal composition.

Among the second component B, preferred examples of the compounds of the formulas (5), (6) and (7) are (5-1) to (5-24), (6-1) to (6-3) and (7-1) to (7-17).

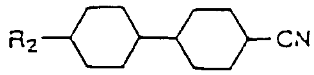
(5-1)

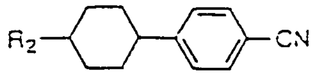
(5-2)

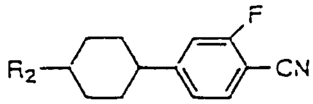
(5-3)

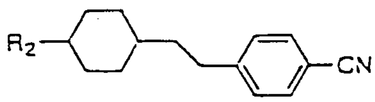
(5-4)

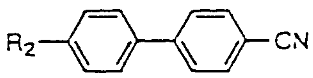
(5-5)

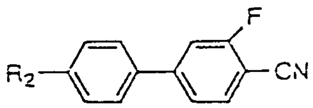
(5-6)

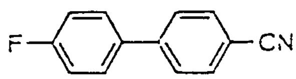
(5-7)

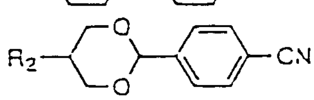
(5-8)

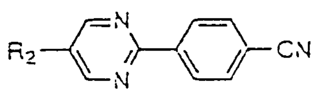
(5-9)

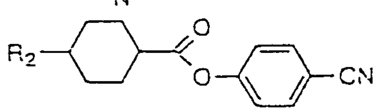
(5-10)

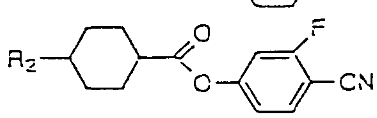
(5-11)

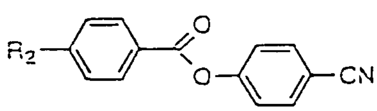
(5-12)

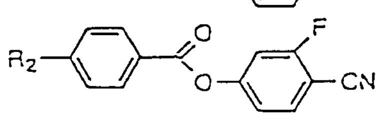
(5-13)

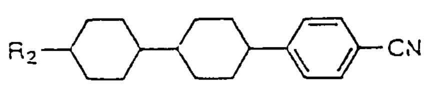
(5-14)

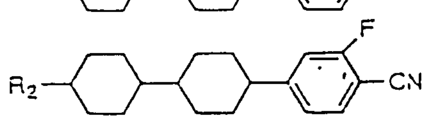
(5-15)

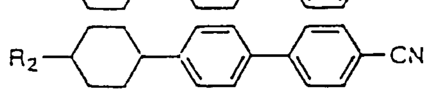
(5-16)

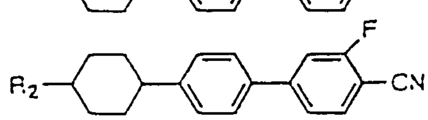
(5-17)

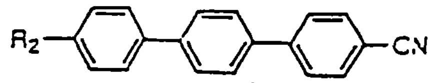

(5-18)

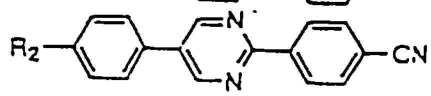

(5-19)

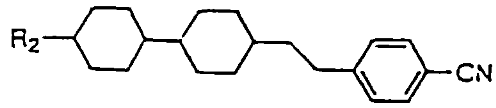

(5-20)

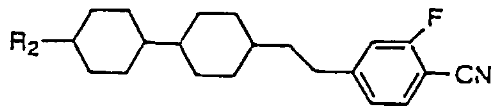

(5-21)

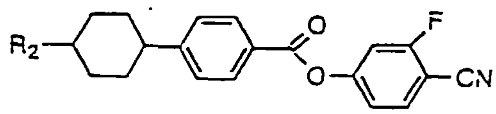

(5-22)

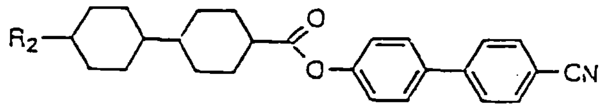

(5-23)

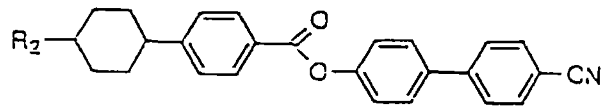

(5-24)

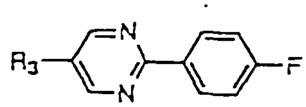

(6-1)

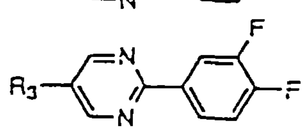

(6-2)

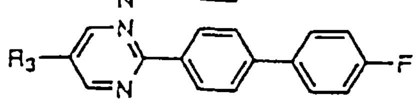

(6-3)

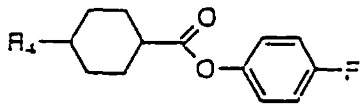

(7-1)

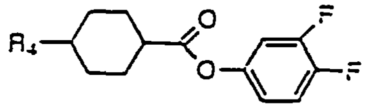

(7-2)

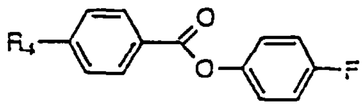

(7-3)

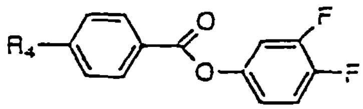

(7-4)

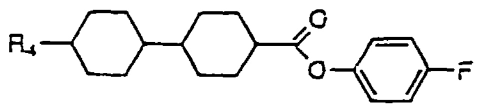

(7-5)

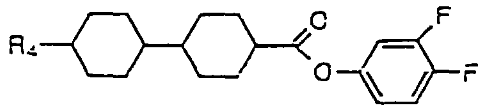

(7-6)

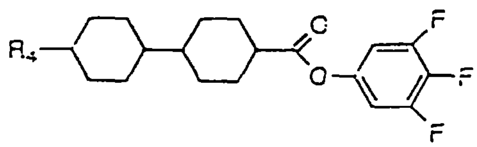

(7-7)

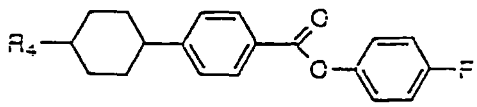

(7-8)

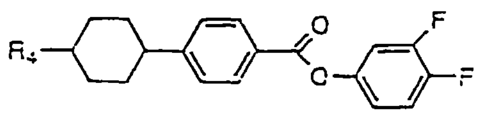

(7-9)

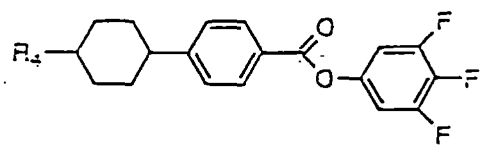

(7-10)

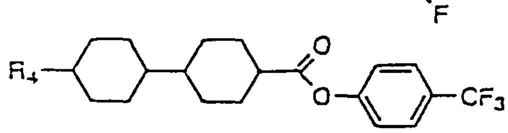

(7-11)

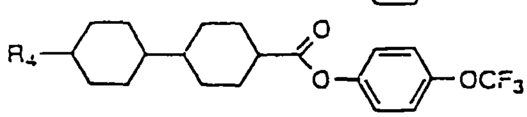

(7-12)

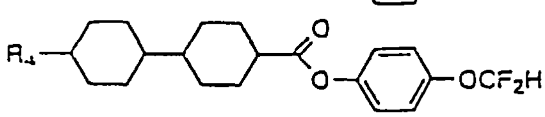

(7-13)

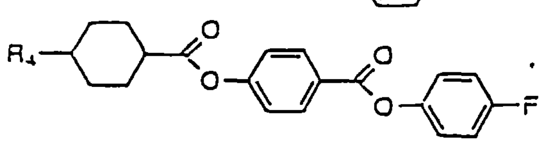

(7-14)

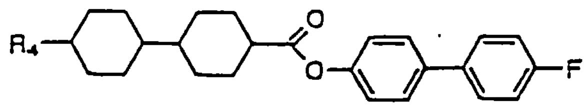
(7-15)

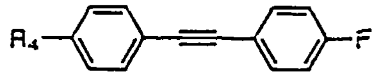
(7-16)

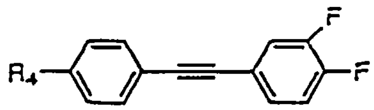
(7-17)

The compounds of the formulas (5) to (7) have large positive dielectric anisotropy and are used for decreasing threshold voltage of the composition. They are also used for adjusting viscosity and optical anisotropy and broadening liquid crystal phase temperature range as well as improving steepness.

Among the second component B, preferred examples of the compounds of the formulas (8) and (9) are (8-1) to (8-8) and (9-1) to (9-12).

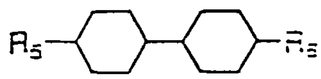
(8-1)

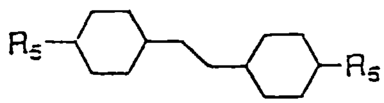
(8-2)

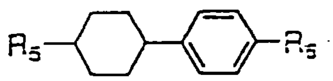
(8-3)

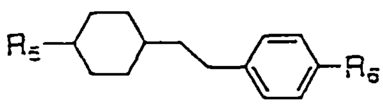
(8-4)

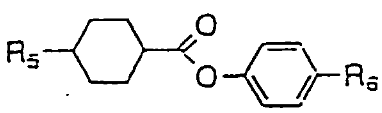
(8-5)

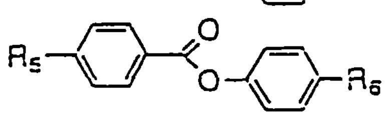
(8-6)

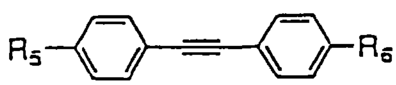
(8-7)

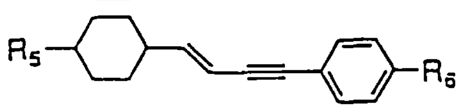
(8-8)

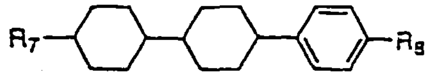

(9-1)

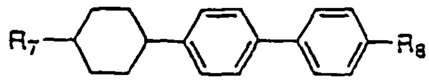

(9-2)

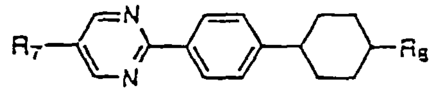

(9-3)

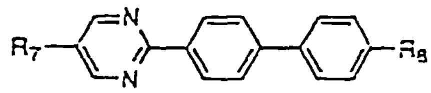

(9-4)

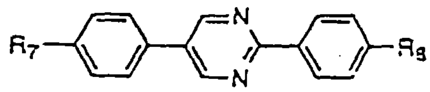

(9-5)

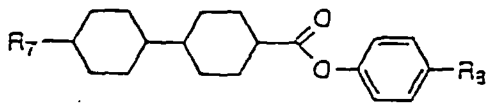

(9-6)

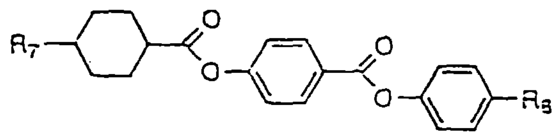

(9-7)

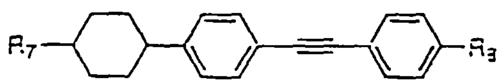

(9-8)

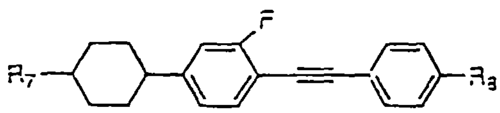

(9-9)

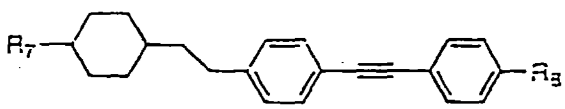

(9-10)

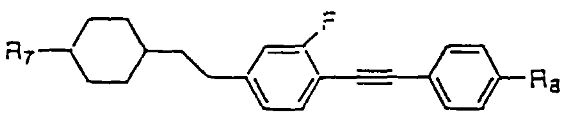

(9-11)

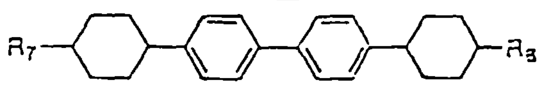

(9-11)

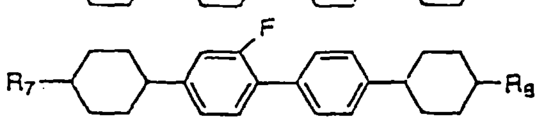

(9-12)

The compounds of the formulas (8) and (9) have negative or weakly positive dielectric anisotropy. The compounds of the formula (8) are used for lowering viscosity or adjusting optical anisotropy, and the compounds of the formula (9) are used for broadening temperature range of liquid crystal phase and/or adjusting optical anisotropy.

The compounds of the formulas (5) to (9) are essential for preparing a liquid crystal composition for STN display system or conventional TN display system. The amount of the compounds used for the preparation of a liquid crystal composition for STN display system or conventional TN display system is suitably 1 to 99 % by weight, preferably 10 to 97 % by weight, more preferably 40 to 95 % by weight based on the total weight of the liquid crystal composition.

The liquid crystal composition of the present invention preferably includes at least one compound of the formula (1) in the amount of 0.1 to 99 % by weight to produce excellent characteristics.

The liquid crystal composition of the present invention can be prepared by conventional methods. Generally, they may be prepared by dissolving various components at an elevated temperature. Further, the liquid crystal composition of the present invention may be improved and optimized by the addition of an additive suitable for an intended purpose.

Such additives are well known in the art and described in detail in literature. See for example "Liquid Crystal Device Handbook" edited by Japan Society for the Promotion of Science, Committee No.142 and published by Nikkan Kogyo Shimbun Sha, pages 192 to 202). Typically, a chiral dopant is added to induce spiral structure of the liquid crystal so as to adjust a desired degree of twist and to restrain reverse twist.

The liquid crystal composition of the present invention can be used as that for guest-host (GH) mode if a dichroic dye such as merocyanine, styryl, azo, azomethine, azoxy, quinophthalone, anthraquinone and tetrazine dyes is added thereto. Further, the composition of the present invention can be used as a liquid crystal composition for polymer dispersed type liquid crystal display element (PDLCD) represented by NCAP (Nematic Curvilinear Aligned Phases) which is obtained by microencapsulating nematic liquid crystal and polymer network liquid crystal display element (PNLCD) which is obtained by forming a three dimensional polymer network in liquid crystal. In addition, the composition of the present invention can also he used as a liquid crystal composition for electrically controlled birefringence (ECB) mode and dynamic scattering (DS) mode.

The following formulations are examples of nematic liquid crystal compositions comprising the compound of the present invention.

Formulation Example 1

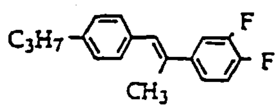 Compound No. 32 8%

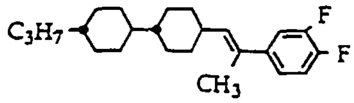 Compound No. 64 12%

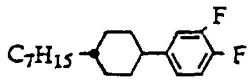 6%

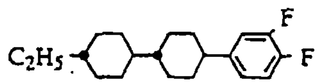 12%

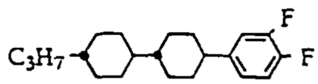 12%

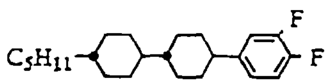 12%

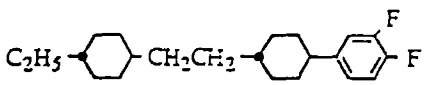 4%

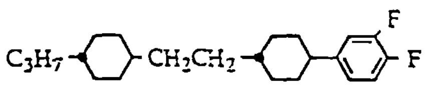 2%

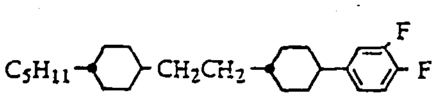 4%

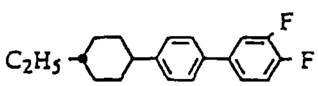 7%

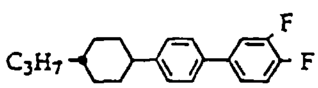 7%

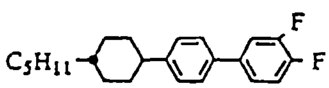 14%

Formulation Example 2

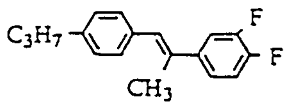 Compound No. 32 5%

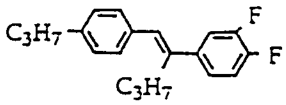 Compound No. 33 3%

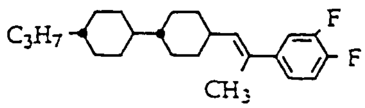 Compound No. 64 10%

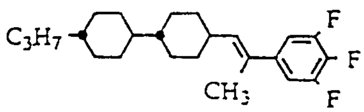 Compound No. 65 8%

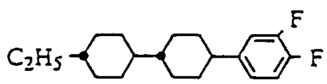 8%

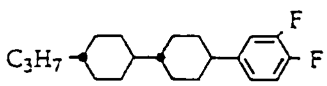 8%

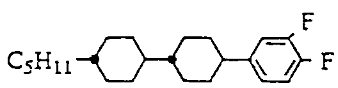 8%

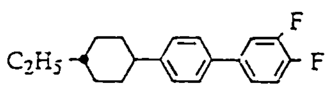 4%

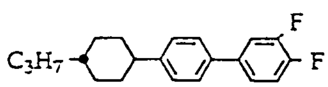 4%

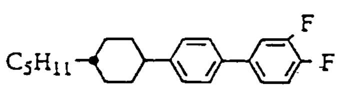 8%

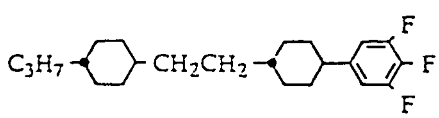 8%

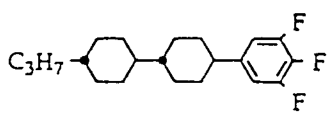 8%

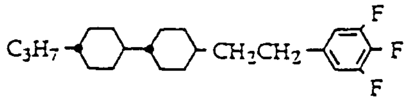 10%

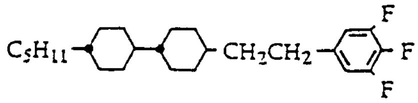 6%

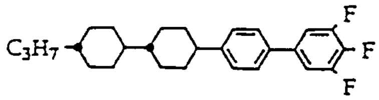 2%

Formulation Example 3

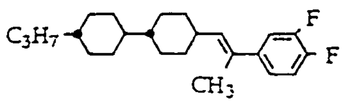 Compound No. 64 8%

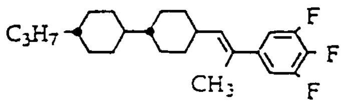 Compound No. 65 10%

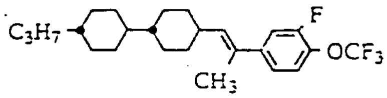 Compound No. 69 5%

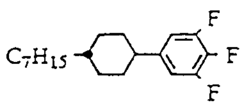 3%

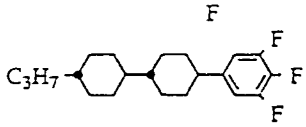 6%

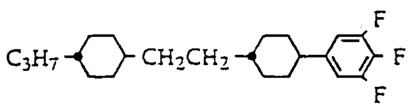 3%

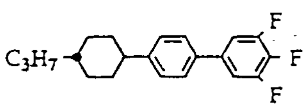 12%

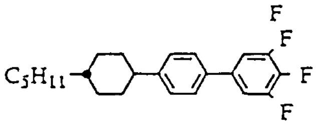 10%

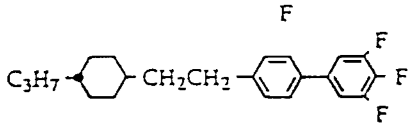 5%

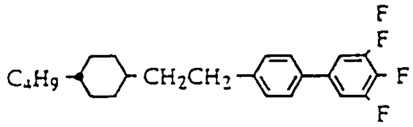 5%

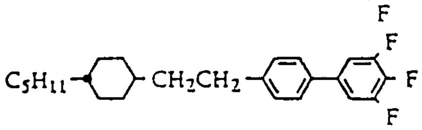 5%

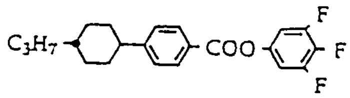 3%

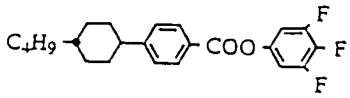 3%

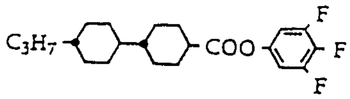 12%

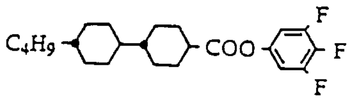 4%

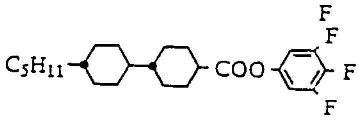 4%

Formulation Example 4

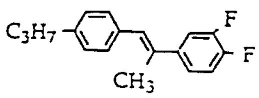 Compound No. 32 4%

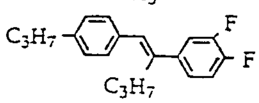 Compound No. 33 3%

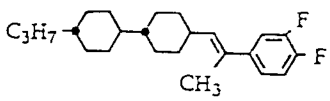 Compound No. 64 15%

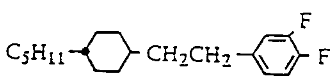 2%

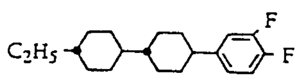 10%

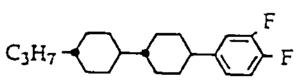 10%

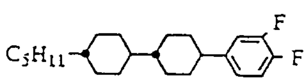 10%

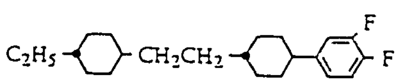 4%

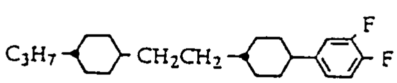 2%

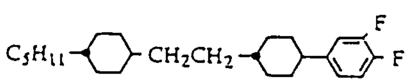 4%

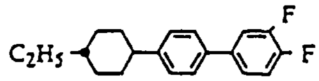

3%

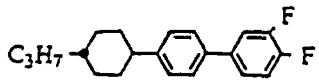

3%

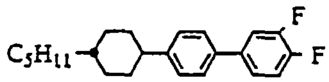

6%

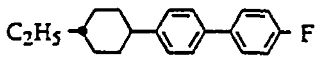

4%

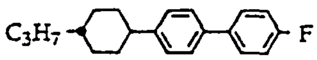

2%

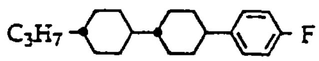

2%

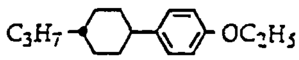

6%

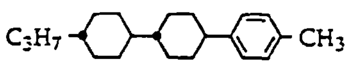

4%

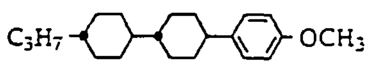

2%

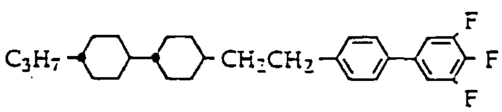

2%

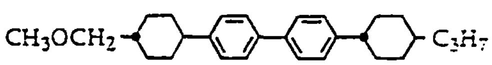

2%

Formulation Example 5

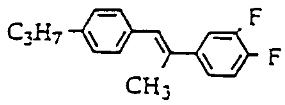 Compound No. 32 5%

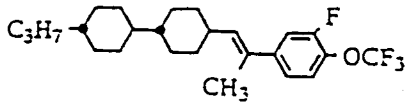 Compound No. 69 5%

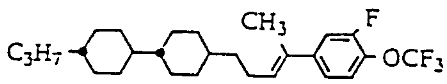 Compound No. 99 4%

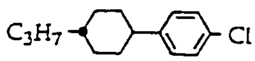 7%

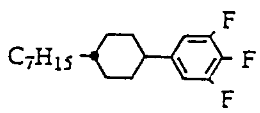 5%

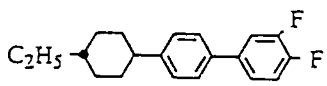 5%

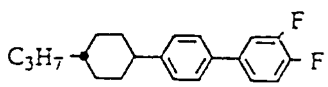 5%

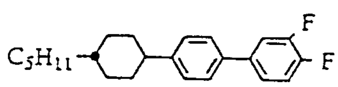 10%

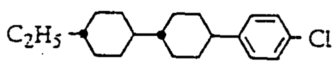 8%

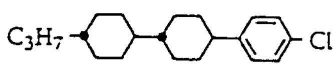 8%

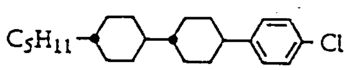 4%

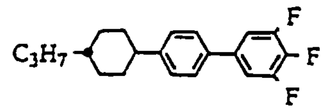

15%

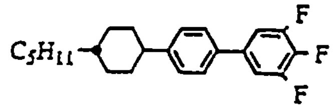

7%

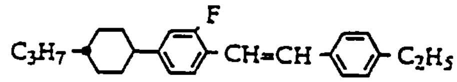

3%

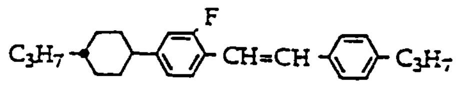

3%

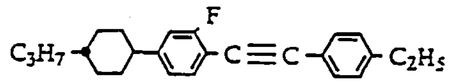

2%

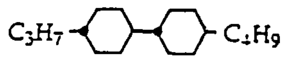

2%

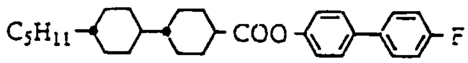

2%

Formulation Example 6

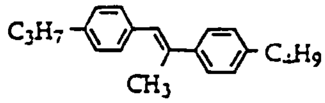 Compound No. 31 7%

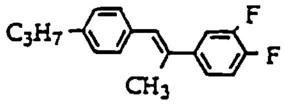 Compound No. 32 10%

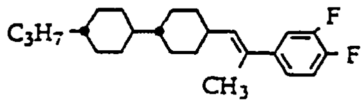 Compound No. 64 9%

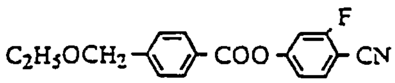 4%

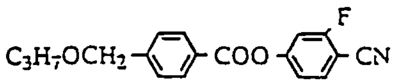 4%

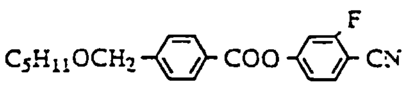 4%

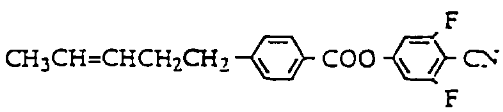 10%

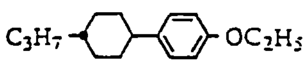 5%

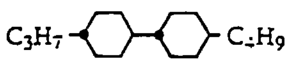 3%

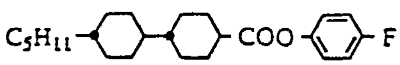 4%

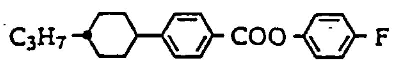 4%

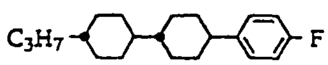 4%

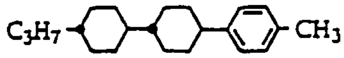 7%

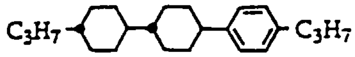 5%

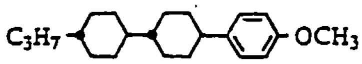 4%

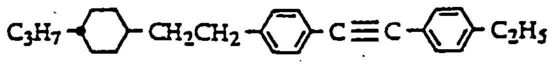 4%

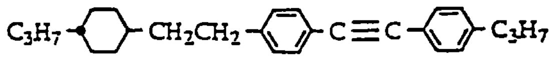 3%

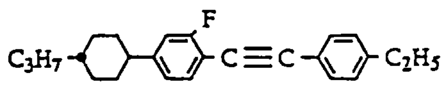 6%

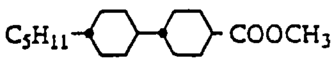 3%

Formulation Example 7

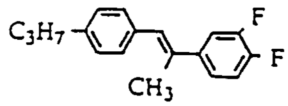 Compound No. 32 6%

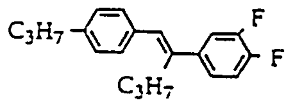 Compound No. 33 5%

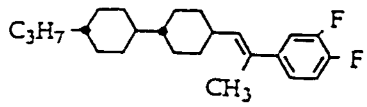 Compound No. 64 11%

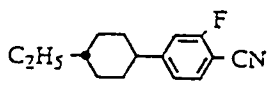 5%

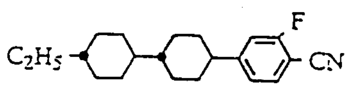 10%

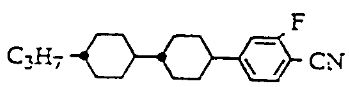 10%

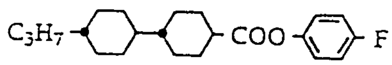 6%

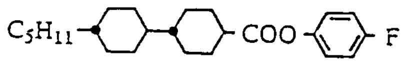 4%

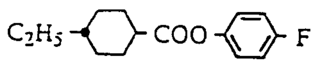 2%

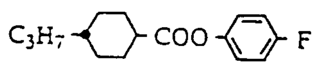 3%

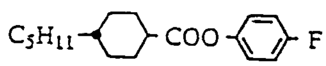 2%

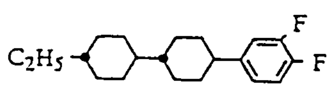 12%

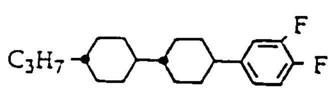 12%

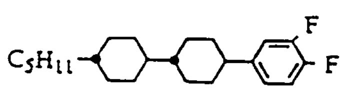 12%

Formulation Example 8

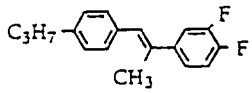 Compound No. 32 5%

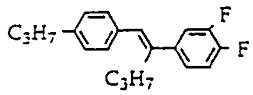 Compound No. 33 5%

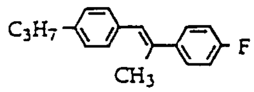 Compound No. 30 8%

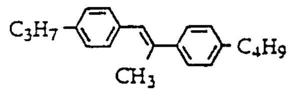 Compound No. 31 7%

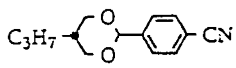 8%

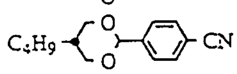 8%

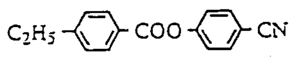 10%

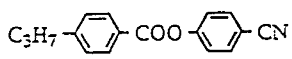 2%

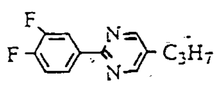 2%

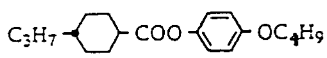 6%

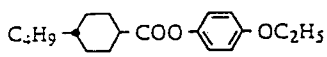 6%

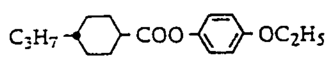 6%

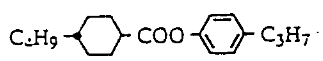 4%

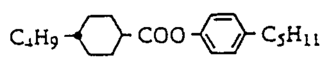 4%

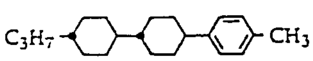 8%

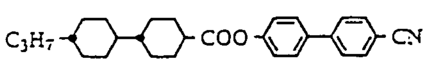 3%

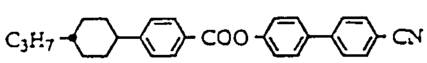 3%

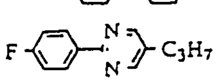 2%

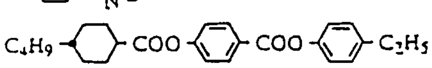 3%

Formulation Example 9

| | | |
|---|---|---|
| 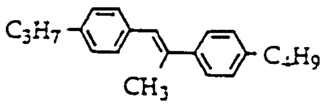 | Compound No. 31 | 7% |
| 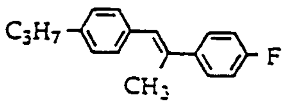 | Compound No. 30 | 6% |
| 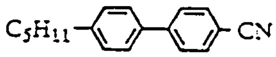 | | 5% |
| 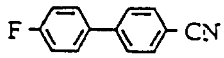 | | 2% |
| 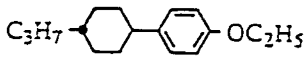 | | 8% |
| 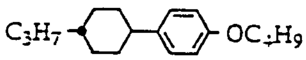 | | 10% |
| 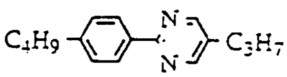 | | 5% |
| 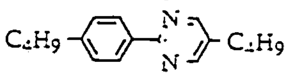 | | 6% |
| 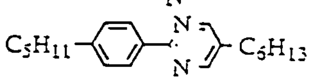 | | 5% |
| 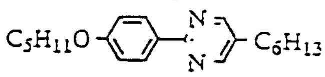 | | 4% |
| 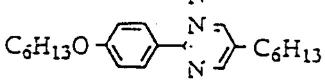 | | 4% |
| 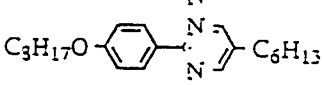 | | 4% |
| 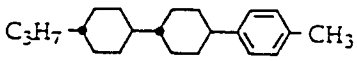 | | 10% |
| 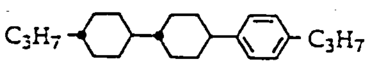 | | 8% |
| 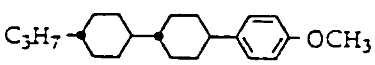 | | 6% |
| 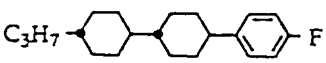 | | 3% |
| 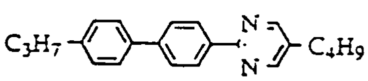 | | 2% |
| 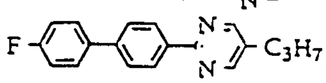 | | 2% |
| 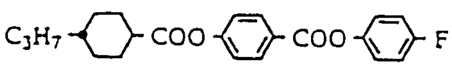 | | 3% |

Formulation Example 10

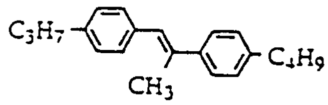 Compound No. 31 5%

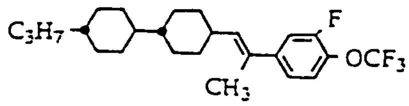 Compound No. 69 8%

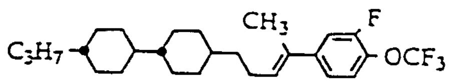 Compound No. 99 8%

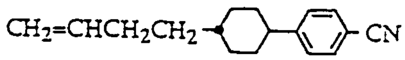 8%

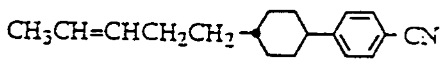 8%

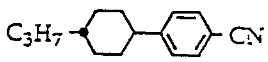 7%

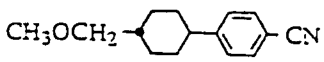 6%

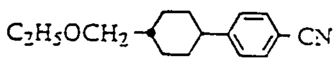 4%

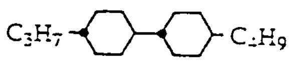 7%

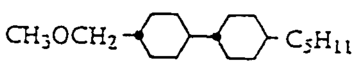 6%

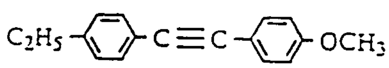 6%

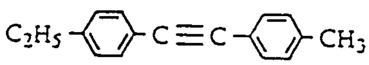 5%

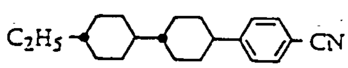 2%

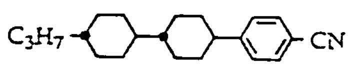 2%

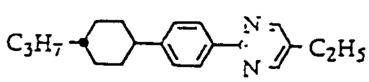 2%

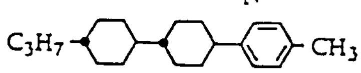 8%

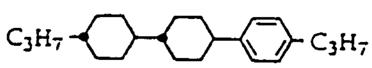 8%

Formulation Example 11

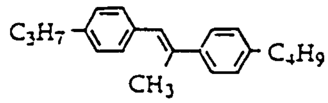 Compound No. 31 5%

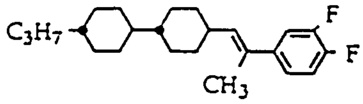 Compound No. 64 10%

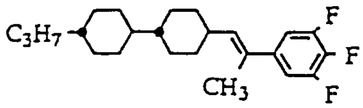 Compound No. 65 5%

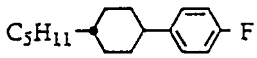 8%

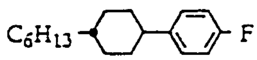 7%

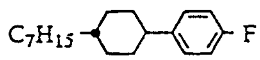 8%

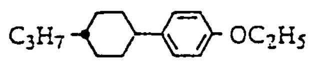 4%

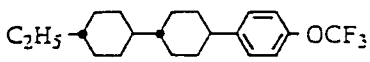 8%

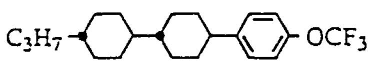 7%

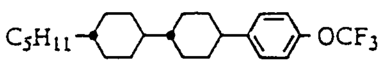 7%

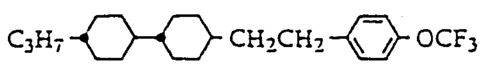 3%

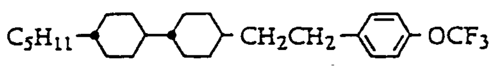 2%

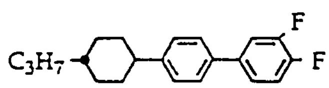 10%

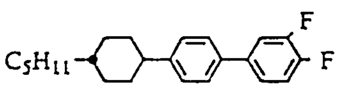 8%

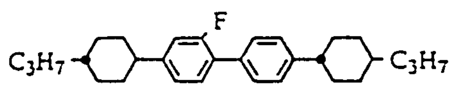 4%

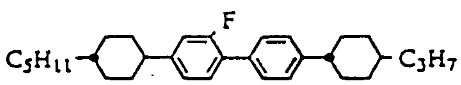 4%

Formulation Example 12

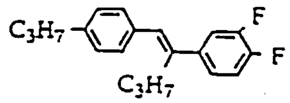
Compound No. 33 8%

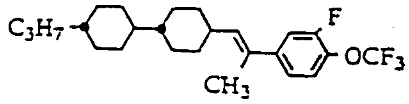
Compound No. 69 8%

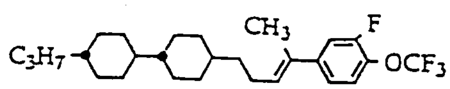
Compound No. 99 4%

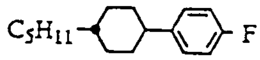
3%

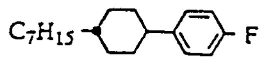
5%

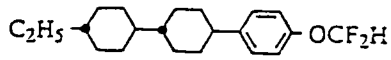
8%

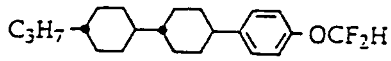
8%

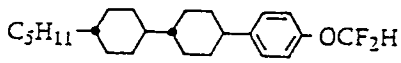
8%

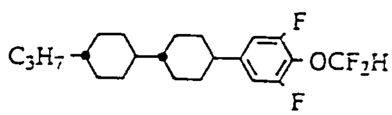
4%

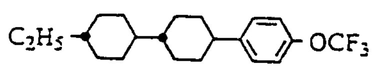
12%

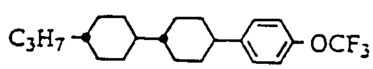
12%

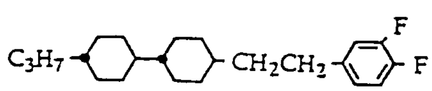
5%

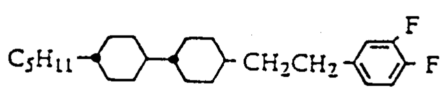
5%

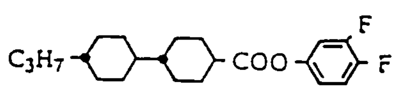
5%

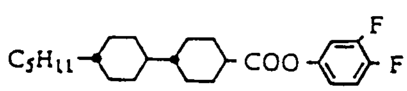
5%

Formulation Example 13

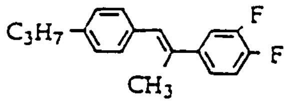 Compound No. 32 4%

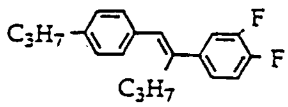 Compound No. 33 4%

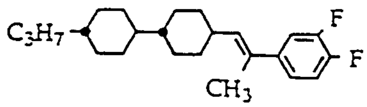 Compound No. 64 12%

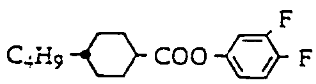 8%

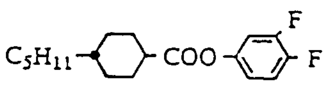 8%

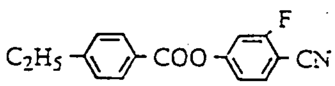 5%

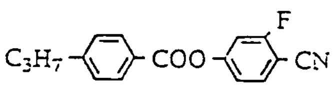 5%

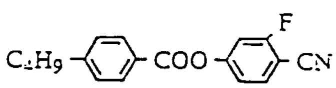 6%

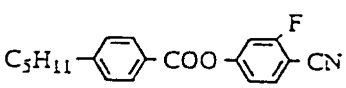 6%

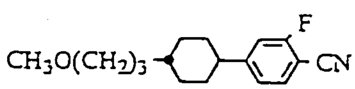 6%

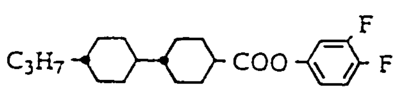 2%

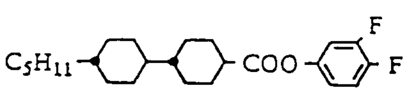 2%

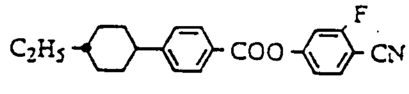 4%

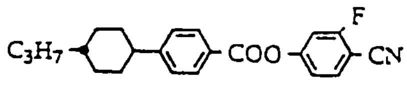 4%

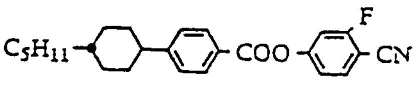 4%

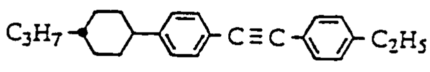 10%

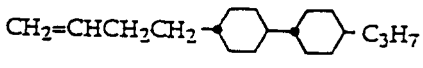 5%

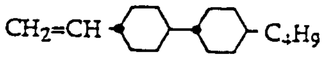 5%

The compounds of the formula (1) of the present invention can easily be prepared by means of conventional methods in organic synthesis, such as those described in "Organic Synthesis", "Organic Reactions" and "Jikken Kagaku Koza". For example, the compounds of the formula (1) wherein $Z_3$ is alkyl-substituted alkenylene Q can be prepared as follows wherein X represents iodine or bromine atom, R, R', rings $A_1$, $A_2$, $A_3$ and $A_4$, $Z_1$, $Z_2$, $P_1$, $P_2$, q, r, m and n are the same as defined above.

Reaction Scheme 1

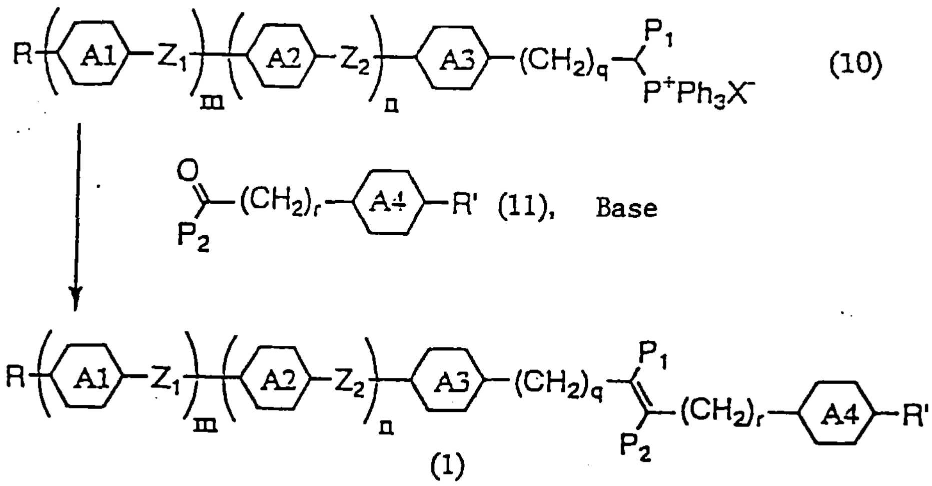


(10)

(1)

The phosphonium salt (10) prepared from corresponding halide according to the process of G.Wittig wt al. Org.Synth.,V,751(1973) is reacted under alkaline condition with the ketone compound (11) according to the process of

D.B.Denny et al., J.Org.Chem.,29,495(1964) to prepare the compound (1) having an olefin moiety. As a base, there can suitably be used alkoxides such as potassium t-butoxide, alkyl lithium such as butyl lithium, alkali metal hydride such as sodium hydride (Reaction Scheme 1). If the olefin moiety produced is cis-form, it can be converted to trans-form by treating with acid such as benzenesulfinic acid. Alternatively, inversion of the olefin moiety may be conducted according to the process described in JP-A-61-83136.

Further, the Grignard reagent (12) prepared from corresponding halide by the process of M.S.Kharasch et al., "Grignard Reactions of Nonmetallic Substances", Prentice-Hall(1954) is reacted with the ketone compound (11) to prepare the alcohol (13), which is then subjected to dehydration under acidic condition to obtain the intended compound (1) (Reaction Scheme 2). Examples of the acids used in the dehydration include mineral acid such as sulfuric acid and hydrochloric acid, sulfonic acid such as toluenesulfonic acid and acidic ion-exchange resin such as Amberlist .

Reaction Scheme 2

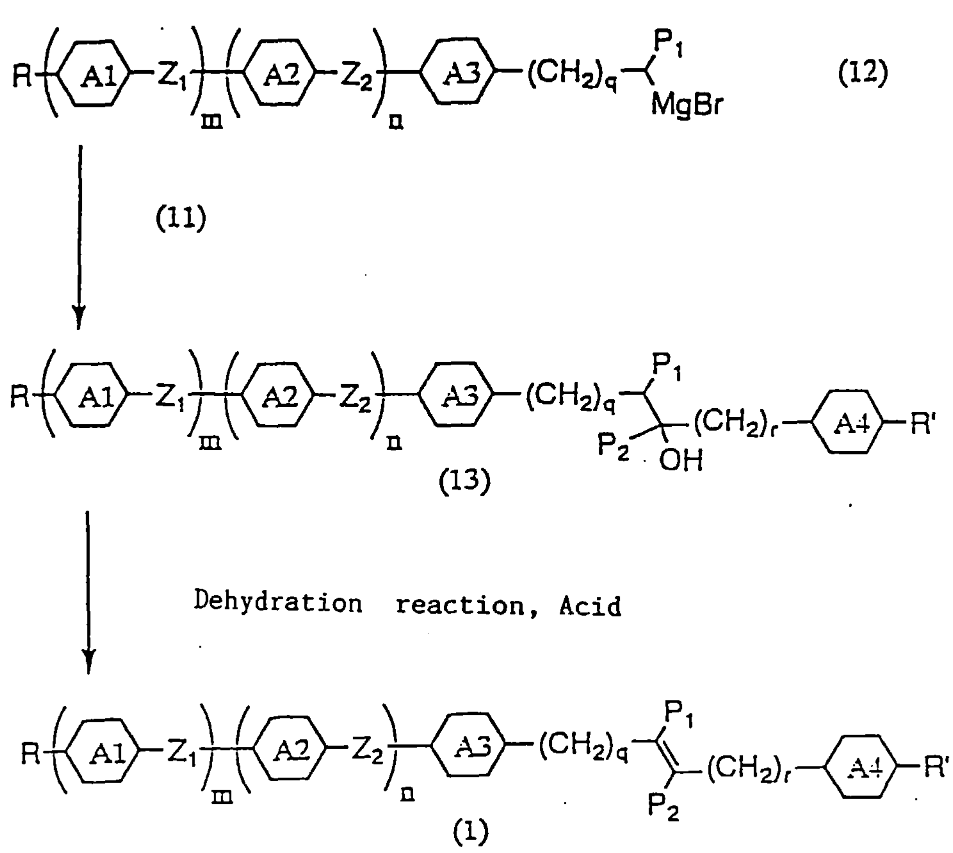


Further, the compounds of the formula (1) can also suitably be prepared by the following route: The carboxylic acid chloride (14) is reacted with the Grignard reagent (15) in the presence of a catalyst according to the process described in WO95/20021 to prepare the ketone compound (16), with which an alkyl Grignard reagent or an alkyl metal compound such as alkyl lithium is reacted according to the process described in H.Gilman et al., Org.Synth.,I,188(1941) or P.D.Bartlett et al., J.Am.Chem.Soc.,77,2804(1955) to prepare the alcohol (17) which is then subjected to dehydration according to Reaction Scheme 2 to obtain the intended compound (1) (Reaction Scheme 3).

Reaction Scheme 3

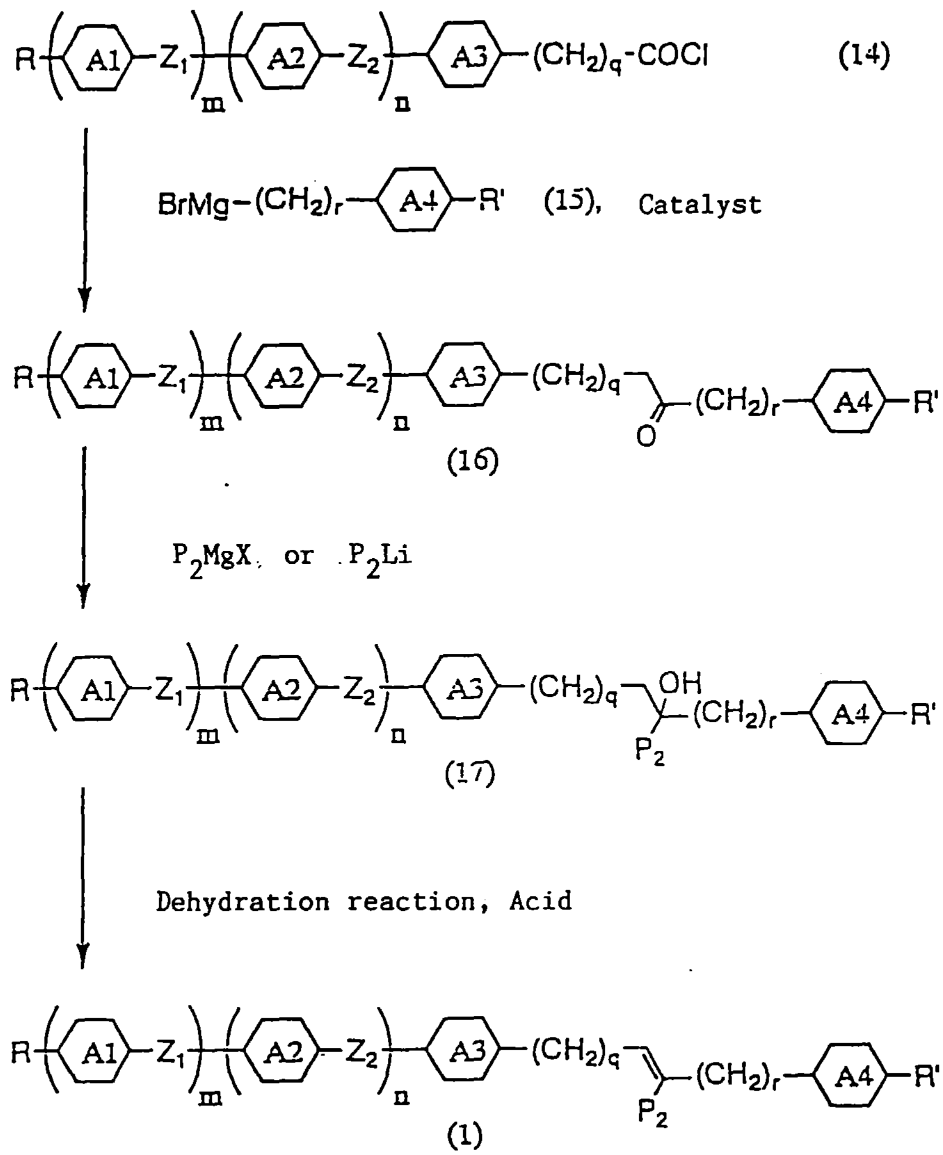


Further, in the similar manner to Reaction Scheme 3, the carboxylic acid chloride (19) and the Grignard reagent (18) are used to prepare a compound of the formula (1) wherein $P_1$ is an alkyl group and $P_2$ is hydrogen atom (Reaction Scheme 4).

Reaction Scheme 4

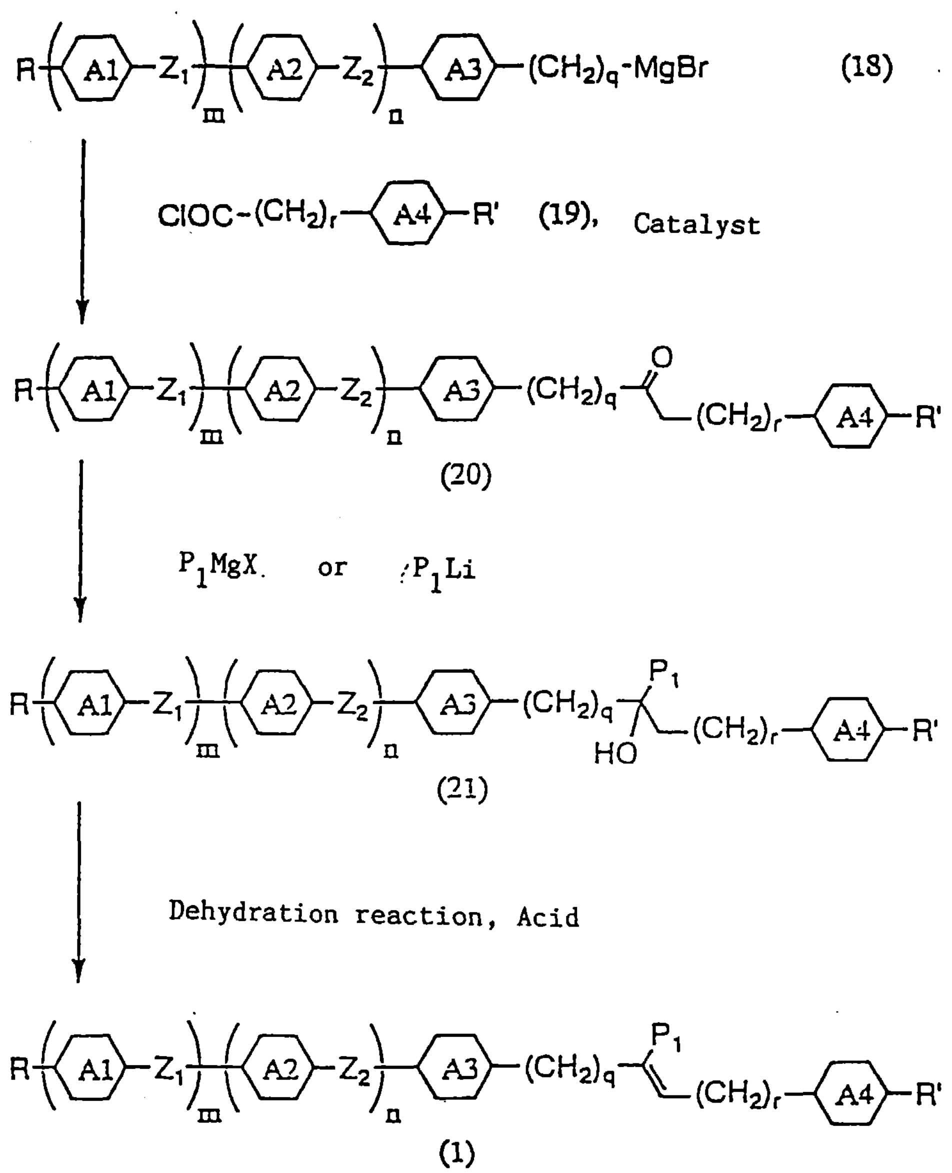


A compound of the formula (1) wherein $Z_2$ is an alkyl-substituted alkenylene Q can be prepared as follows.

The alcohol prepared according to the process described in WO95/20021 is transformed into the halide (22) according to the process described in for example, D.K.Black et al., Tetrahedron Lett.,483(1963) and J.P.Schaeffer et al., Org.Synth., V,249(1973). The halide (22) is reacted with triphenyl phosphine or magnesium in the same manner as described earlier to prepare the corresponding phosphonium salt (23) or the Grignard reagent (24), respectively, which is then reacted with the ketone compound (25) according to Reaction Schemes 1 and 2 to obtain the compound (1) (Reaction Scheme 5).

Reaction Scheme 5

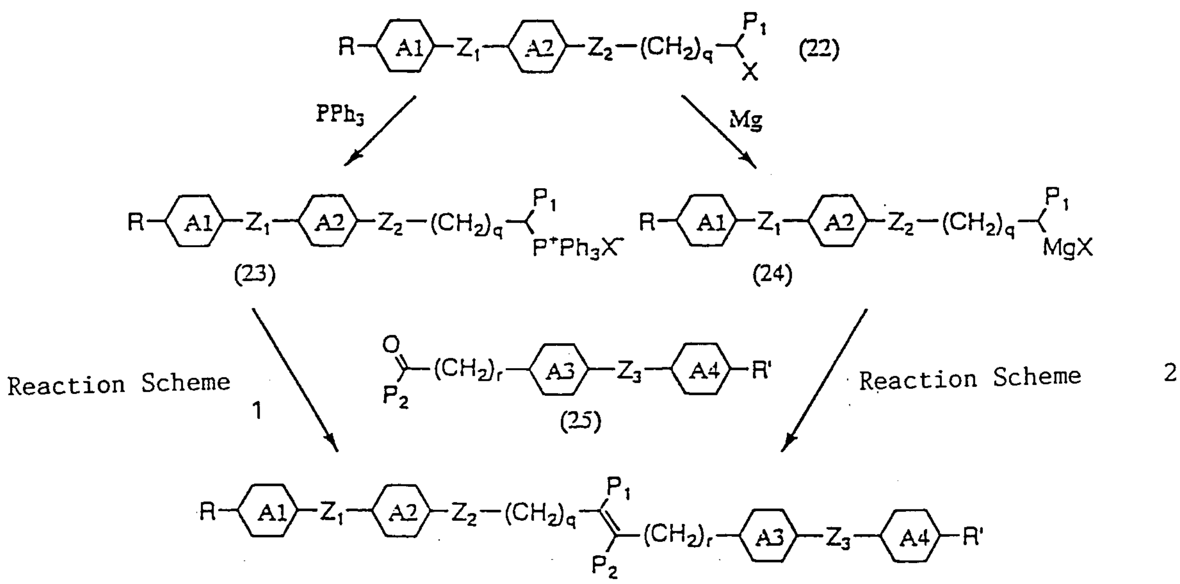


(1)

## Examples

The following examples explain in detail the method for the preparation of the compounds of the present invention and use thereof. In the examples, C means crystal, N means nematic phase, S means smectic phase, and I means isotropic liquid. The unit of phase transition temperature is °C.

### Example 1

Preparation of 4-(1-methyl-2-(4-(4-propylcyclohexyl) cyclohexyl) ethenyl)-1,2-difluorobenzene

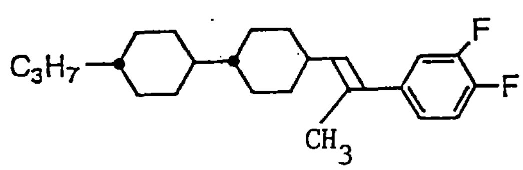

(The compound of the formula (1) wherein R is propyl, rings $A_2$ and $A_3$ are 1,4-cyclohexane rings, ring $A_4$ is 3-fluorol,4-phenylene ring, $Z_2$ is a covalent bond, $Z_3$ is Q , m is 0, n is 1, R ' is fluorine atom, $P_1$ is hydrogen atom, $P_2$ is methyl, and q = r = 0 , Compound No.64)

(The first step)

A mixture of 4-(4-propylcyclohexyl)cyclohexyl acetic acid (90 mmol) and thionyl chloride (450 mmol) was heated and stirred under reflux for 3 hours. After cooled, excess thionyl chloride was distilled off to obtain, as residue, light yellow solid of 4-(4-propylcyclohexyl) cyclohexyl acetic acid chloride (90 mmol, yield 100 %).

(The second step)

To a mixture of fully dried metal magnesium (99 mmol) and THF (20 ml), there was dropped 3,4-difluorobromobenzene (100 mmol) in THF (150 ml) solution at a temperature of 50°C or lower to produce a Grignard reagent. The mixture was stirred for additional two hours at room temperature to confirm that metal magnesium was not remained.

(The third step)

To a mixture of 4-(4-propylcyclohexyl)cyclohexyl acetic acid chloride (90 mmol), dried toluene (500 ml), and iron acetylacetone (0.9 mmol), there was dropped the Grignard reagent prepared in the second step at a temperature of -60°C or lower to obtain a black solution. The mixture was stirred for 30 minutes at the same temperature and then poured into 6M HCl (500 ml) and stirred sufficiently. The reaction solution was transferred to a separating funnel. The organic layer separated was washed with 500 ml of 6M HCl twice and 500 ml of water thrice and dried on anhydrous magnesium sulfate. The solvent was distilled off under reduced pressure. The residue was purified by distillation under reduced pressure and recrystallization from ethanol (50 ml) to obtain 4-(4-(2-oxo-2-(3,4-difluorophenyl)ethyl)cyclohexcyl)-1-propylcyclohexane (62 mmol, yield 69 %).

(The fourth step)

To a solution of 4-(4-(2-oxo-2-(3,4-difluorophenyl)ethyl) cyclohexcyl)-1-propylcyclohexane (62 mmol) obtained in the third step in 300 ml of diethyl ether, there was dropped a solution of magnesium methyl iodide in diethyl ether (2M solution, corresponding to 74 mmol) at a temperature of 20 °C or lower and stirred at room temperature for one hour. To the reaction mixture, 6M HCl (20 ml) was slowly added. The mixture was transferred to a separating funnel and stirred sufficiently. The organic layer separated was dried on anhydrous magnesium sulfate and diethyl ether was distilled under reduced pressure. To the residue, toluene (50 ml) and p-toluenesulfonic acid monohydrate (6.2 mmol) were added and heated under reflux for four hours while removing water formed.

After cooled, the reaction mixture was transferred to a separating funnel and washed with water sufficiently and dried on anhydrous magnesium sulfate. The solvent was distilled under reduced pressure and the resulting residue was purified by silica gel chromatography (eluate: heptane) followed by recrystallization (twice from 30 ml of ethanol) to obtain the title compound (32 mmol, yield 51 %).

The compound has a liquid crystal phase and transition temperature: C-N point = 56°C , N-I point = 120 °C . Various spectral data fully supported the structure of the compound.

Example 2

In the similar manner to those of Example 1, the following compounds were prepared. The compound prepared in Example 1 is also shown.

Compound No.1
4-(2-methyl-2-(4-methylcyclohexyl)ethenyl)-1-propylcyclohexane
Compound No.2
4-(2-methyl-2-(4-methylcyclohexyl)ethenyl)-1-ethoxymethylcyclohexane
Compound No.3
4-(2-methyl-2-(4-propylcyclohexyl)ethenyl)-1-propylcyclohexane
Compound No.4
4-(2-methyl-2-(4-propylcyclohexyl)ethenyl)-1-butylcyclohexane
Compound No.5
4-(2-methyl-2-(4-propylcyclohexyl)ethenyl)-1-vinylcyclohexane
Compound No.6
4-(2-methyl-2-(4-vinylcyclohexyl)ethenyl)-1-vinylcyclohexane
Compound No.7
4-(2-methyl-2-(4-propylcyclohexyl)ethenyl)-1-(3-butenyl)cyclohexane
Compound No.8
4-(1,2-dimethyl-2-(4-propylcyclohexyl)ethenyl)-1-butylcyclohexane
Compound No.9
4-(2-ethyl-2-(4-propylcyclohexyl)ethenyl)-1-butylcyclohexane
Compound No.10
4-(2-propyl-2-(4-propylcyclohexyl)ethenyl)-1-butylcyclohexane

Compound No.11
4-(2-methyl-2-(4-methylphenyl)ethenyl)-1-propylcyclohexane
Compound No.12
4-(2-methyl-2-(4-propylphenyl)ethenyl)-1-propylcyclohexane
Compound No.13
4-(2-methyl-2-(4-propylphenyl)ethenyl)-1-butylcyclohexane
Compound No.14
4-(1-methyl-2-(4-propylphenyl)ethenyl)-1-butylcyclohexane
Compound No.15
4-(2-methyl-2-(4-fluorophenyl)ethenyl)-1-propylcyclohexane
Compound No.16
4-(2-methyl-2-(3,4-difluorophenyl)ethenyl)-1-propylcyclohexane
Compound No.17
4-(2-methyl-2-(3,4-difluorophenyl)ethenyl)-1-pentylcyclohexane
Compound No.18
4-(2-methyl-2-(4-cyanophenyl)ethenyl)-1-pentylcyclohexane
Compound No.19
4-(2-methyl-2-(4-trifluoromethoxyphenyl)ethenyl)-1-propylcyclohexane
Compound No.20
4-(2-methyl-2-(4-trifluoromethoxyphenyl)ethenyl)-1-pentylcyclohexane
Compound No.21
4-(2-methyl-2-(3-fluoro-4-trifluoromethoxyphenyl)ethenyl)-1-propylcyclohexane
Compound No.22
4-(2-methyl-2-(3-fluoro-4-trifluoromethoxyphenyl)ethenyl)-1-propyloxymethylcyclohexane
Compound No.23
4-(2-methyl-2-(4-trifluoromethylphenyl)ethenyl)-1-propylcyclohexane
Compound No.24
4-(2-methyl-2-(4-trifluoromethylphenyl)ethenyl)-1-pentylcyclohexane
Compound No.25
4-(2-methyl-2-(4-trifluoromethylphenyl)ethenyl)-1-propylcyclohexane
Compound No.26
4-(1-methyl-2-(4-trifluoromethylphenyl)ethenyl)-1-propylcyclohexane
Compound No.27
4-(2-methyl-2-(4-propylphenyl)ethenyl)-1-propylbenzene
Compound No.28
4-(2-methyl-2-(4-propylphenyl)ethenyl)-1-butylbenzene
Compound No.29
4-(2-propyl-2-(4-propylphenyl)ethenyl)-1-butylbenzene
Compound No.30
4-(2-methyl-2-(4-propylphenyl)ethenyl)-1-fluorobenzene
Compound No.31
4-(1-methyl-2-(4-propylphenyl)ethenyl)-1-butylbenzene
mp : 29.4°C
Compound No.32
4-(1-methyl-2-(4-propylphenyl)ethenyl)-1,2-difluorobenzene
mp : 40.5°C
Compound No.33
4-(1-propyl-2-(4-propylphenyl)ethenyl)-1,2-difluorobenzene
Compound No.34
4-(1-methyl-2-(4-propylphenyl)ethenyl)-1-cyanobenzene
Compound No.35
4-(1-methyl-2-(4-propylphenyl)ethenyl)-1-cyano-2-fluorobenzene
Compound No.36
4-(1-methyl-2-(4-methoxymethylphenyl)ethenyl)-1-cyano-2-fluorobenzene
Compound No.37
4-(1-methyl-2-(4-propylphenyl)ethenyl)-1-cyano-2,6-difluorobenzene
Compound No.38
4-(1-methyl-2-(4-vinylphenyl)ethenyl)-1-cyano-2,6-difluorobenzene

Compound No.39
4-(1-methyl-2-(4-(3-pentenylphenyl)ethenyl)-1-cyano-2,6-difluorobenzene
Compound No.40
4-(1-methyl-2-(4-(3-pentynylphenyl)ethenyl)-1-cyano-2,6-difluorobenzene
Compound No.41
4-(1-methyl-2-(4-propylphenyl)ethenyl)-1-trifluoromethoxybenzene
Compound No.42
4-(1-methyl-2-(4-propylphenyl)ethenyl)-1-trifluoromethoxy-2-fluorobenzene
Compound No.43
4-(1-propyl-2-(4-propylphenyl)ethenyl)-1-trifluoromethoxy-2-fluorobenzene
Compound No.44
4-(1-methyl-2-(4-propylphenyl)ethenyl)-1-trifluoromethylbenzene
Compound No.45
4-(1-methyl-2-(4-propylphenyl)ethenyl)-1-trifluoromethyl-2,6-difluorobenzene
Compound No.46
4-(1-methyl-2-(4-propylphenyl)ethenyl)-1-(1,1,2,2-tetrafluoroethoxy) benzene
Compound No.47
4-(1-methyl-2-(4-propylphenyl)ethenyl)-1-(1,1,2,3,3,3-hexafluoro ethoxy)benzene
Compound No.48
4-(4-methyl-4-(4-propylcyclohexyl)-3-butenyl)-1-methylcyclohexane
Compound No.49
4-(4-methyl-4-(4-propylcyclohexyl)-3-butenyl)-1-propylcyclohexane
Compound No.50
4-(3-methyl-4-(4-propylcyclohexyl)-3-butenyl)-1-methylcyclohexane
Compound No.51
4-(3-methyl-4-(4-propylcyclohexyl)-2-butenyl)-1-methylcyclohexane
Compound No.52
4-(4-methyl-4-(4-trifluoromethoxyphenyl)-3-butenyl)-1-propylcyclo hexane
Compound No.53
4-(2-methyl-4-(4-trifluoromethoxyphenyl)-1-butenyl)-1-propylcyclo hexane
Compound No.54
4-(1-methyl-4-(4-propylphenyl)-1-butenyl)-1-trifluoromethylbenzene
Compound No.55
4-(1-methyl-2-(4-(4-propylcyclohexyl)cyclohexyl)ethenyl)-1-propyl cyclohexane
Compound No.56
4-(1-methyl-2-(4-(4-(3-butenyl)cyclohexyl)cyclohexyl)ethenyl)-1-propylcyclohexane
Compound No.57
4-(2-methyl-2-(4-(4-propylcyclohexyl)cyclohexyl)ethenyl)-1-propyl cyclohexane
Compound No.58
4-(2-methyl-2-(4-(4-(3-pentenyl)cyclohexyl)cyclohexyl)ethenyl)-1-propylcyclohexane
Compound No.59
4-(2-methyl-2-(4-(2-(4-propylcyclohexyl)ethyl)cyclohexyl)ethenyl)-1-propylcyclohexane
Compound No.60
4-(2-methyl-2-(4-(2-(4-propylcyclohexyl)ethyl)cyclohexyl)ethenyl)-1-pentylcyclohexane
Compound No.61
4-(1-methyl-2-(4-(4-propylcyclohexylcarbonyloxy)cyclohexyl)ethenyl)-1-propylbenzene
Compound No.62
4-(1-methyl-2-(4-(4-propylcyclohexyl)cyclohexyl)ethenyl)-1-fluorobenzene
Compound No.63
4-(1-methyl-2-(4-(4-pentylcyclohexyl)cyclohexyl)ethenyl)-1-fluorobenzene
Compound No.64
4-(1-methyl-2-(4-(4-propylcyclohexyl)cyclohexyl)ethenyl)-1,2-difluorobenzene
C-Npoint 56°C, N-I point 120°C
Compound No.65
4-(1-methyl-2-(4-(4-propylcyclohexyl)cyclohexyl)ethenyl)-1,2,6-trifluorobenzene
C-Npoint 58.5°C, N-I point 99.8°C
Compound No.66
4-(1-methyl-2-(4-(4-propylcyclohexyl)cyclohexyl)ethenyl)-1-trifluoromethoxybenzene

Compound No.67
4-(1-methyl-2-(4-(4-pentylcyclohexyl)cyclohexyl)ethenyl)-1-trifluoromethoxybenzene
Compound No.68
4-(1-methyl-2-(4-(4-methoxymethylcyclohexyl)cyclohexyl)ethenyl)-1-trifluoromethoxybenzene
Compound No.69
4-(1-methyl-2-(4-(4-propylcyclohexyl)cyclohexyl)ethenyl)-1-trifluoromethoxy-2-fluorobenzene
Compound No.70
4-(1-methyl-2-(4-(4-propylcyclohexyl)cyclohexyl)ethenyl)-1-trifluoromethylbenzene
Compound No.71
4-(1-methyl-2-(4-(4-propylcyclohexyl)cyclohexyl)ethenyl)-1-trifluoromethyl-2,6-difluorobenzene
Compound No.72
4-(1-methyl-2-(4-(4-pentylcyclohexyl)cyclohexyl)ethenyl)-1-trifluoromethyl-2,6-difluorobenzene
Compound No.73
4-(1-methyl-2-(4-(2-(4-propylcyclohexyl)ethyl)cyclohexyl)ethenyl)-1-trifluoromethyl-2,6-difluorobenzene
Compound No.74
4-(1-methyl-2-(4-(4-propylcyclohexyl)phenyl)ethenyl)-1-propylbenzene
Compound No.75
4-(1-methyl-2-(4-(4-propylcyclohexyl)phenyl)ethenyl)-1-pentylbenzene
Compound No.76
4-(1-methyl-2-(4-(4-propylcyclohexyl)phenyl)ethenyl)-1-fluorobenzene
Compound No.77
4-(1-methyl-2-(4-(4-propylcyclohexyl)phenyl)ethenyl)-1,2-difluoro benzene
Compound No.78
4-(1-methyl-2-(4-(4-propylcyclohexyl)phenyl)ethenyl)-1,2,6-trifluoro benzene
Compound No.79
4-(1-methyl-2-(4-(4-propylcyclohexyl)phenyl)ethenyl)-1-trifluoro methoxybenzene
Compound No.80
4-(1-methyl-2-(2-fluoro-4-(4-propylcyclohexyl)phenyl)ethenyl)-1-trifluoromethoxy-2-fluorobenzene
Compound No.81
4-(1-methyl-2-(4-(4-propylcyclohexyl)phenyl)ethenyl)-1-trifluoromethylbenzene
Compound No.82
4-(1-methyl-2-(4-(4-pentylcyclohexyl)phenyl)ethenyl)-1-trifluoromethylbenzene
Compound No.83
4-(1-methyl-2-(4-(4-(3-butenyl)cyclohexyl)phenyl)ethenyl)-1-trifluoro methylbenzene
Compound No.84
4-(1-methyl-2-(4-(4-(3-pentenyl)cyclohexyl)phenyl)ethenyl)-1-trifluoromethylbenzene
Compound No.85
4-(1-methyl-2-(4-(4-(3-pentynyl)cyclohexyl)phenyl)ethenyl)-1-trifluoromethylbenzene
Compound No.86
4-(1-methyl-2-(4-(4-methoxymethylcyclohexyl)phenyl)ethenyl)-1-trifluoromethylbenzene
Compound No.87
4-(1-methyl-2-(4-(4-propylcyclohexyl)phenyl)ethenyl)-1-trifluoro methyl-2,6-difluorobenzene
Compound No.88
4-(1-methyl-2-(4-(4-pentylcyclohexyl)phenyl)ethenyl)-1-trifluoro methyl-2,6-difluorobenzene
Compound No.89
4-(1-methyl-2-(4-(4-propoxymethylcyclohexyl)phenyl)ethenyl)-1-trifluoromethyl-2,6-difluorobenzene
Compound No.90
4-(1-methyl-2-(4-(2-(4-pentylphenyl)ethynyl)phenyl)ethenyl)-1-pentyl benzene
Compound No.91
4-(1-methyl-2-(4-(4-propylphenyldifluoromethoxy)phenyl)ethenyl)-1-trifluoromethoxy-2-fluorobenzene
Compound No.92
4-(1-methyl-4-(4-(4-propylcyclohexyl)cyclohexyl)-1-butenyl)-1-propyl cyclohexane
Compound No.93
4-(1-methyl-4-(4-(4-propylcyclohexyl)cyclohexyl)-1-butenyl)-1,2-difluorobenzene
Compound No.94
4-(1-methyl-4-(4-(4-butylcyclohexyl)cyclohexyl)-1-butenyl)-1,2-difluorobenzene
Compound No.95
4-(1-methyl-4-(4-(4-pentylcyclohexyl)cyclohexyl)-1-butenyl)-1,2-difluorobenzene

Compound No.96
4-(1-methyl-4-(4-(4-propylcyclohexyl)cyclohexyl)-1-butenyl)-1,2,6-trifluorobenzene
Compound No.97
4-(1-methyl-4-(4-(4-butylcyclohexyl)cyclohexyl)-1-butenyl)-1,2,6-trifluorobenzene
Compound No.98
4-(1-methyl-4-(4-(4-pentylcyclohexyl)cyclohexyl)-1-butenyl)-1,2,6-trifluorobenzene
Compound No.99
4-(1-methyl-4-(4-(4-propylcyclohexyl)cyclohexyl)-1-butenyl)-1-trifluoromethoxy-2-fluorobenzene
Compound No.100
4-(4-methyl-4-(4-(4-propylcyclohexyl)cyclohexyl)-3-butenyl)-1-trifluoromethoxy-2-fluorobenzene
Compound No.101
4-(1-methyl-4-(4-(4-propylcyclohexyl)cyclohexyl)-1-butenyl)-1-trifluoromethyl-2,6-difluorobenzene
Compound No.102
4-(1-methyl-4-(4-(4-propylcyclohexyl)phenyl)-1-butenyl)-1-trifluoro methoxy-2-fluorobenzene
Compound No.103
4-(1-methyl-4-(4-(4-pentylcyclohexyl)phenyl)-1-butenyl)-1-trifluoro methoxy-2-fluorobenzene
Compound No.104
4-(1-methyl-4-(4-(4-propylcyclohexyl)phenyl)-1-butenyl)-1-trifluoro methyl-2-fluorobenzene
Compound No.105
4-(1-methyl-4-(2-fluoro-4-(4-pentylcyclohexyl)phenyl)-1-butenyl)-1-trifluoromethyl-2-fluorobenzene
Compound No.106
4-(1-methyl-4-(2-fluoro-4-(4-methoxymethylcyclohexyl)phenyl)-1-butenyl)-1-trifluoromethyl-2-fluorobenzene
Compound No.107
4-(1-methyl-2-(4-(4-(4-propylcyclohexyl)cyclohexyl)phenyl)ethenyl)-1,2-difluorobenzene
Compound No.108
4-(1-methyl-2-(4-(4-(4-pentylcyclohexyl)cyclohexyl)phenyl)ethenyl)-1,2-difluorobenzene
Compound No.109
4-(1-methyl-2-(4-(4-(4-methoxymethylcyclohexyl)cyclohexyl)phenyl) ethenyl)-1,2-difluorobenzene
Compound No.110
4-(1-methyl-2-(4-(4-(4-propylcyclohexyl)cyclohexyl)phenyl)ethenyl)-1,2,6-trifluorobenzene
Compound No.111
4-(1-methyl-2-(4-(4-(4-butylcyclohexyl)cyclohexyl)phenyl)ethenyl)-1,2,6-trifluorobenzene
Compound No.112
4-(1-methyl-2-(4-(4-(4-pentylcyclohexyl)cyclohexyl)phenyl)ethenyl)-1,2,6-trifluorobenzene
Compound No.113
4-(1-methyl-2-(4-(4-(4-propylcyclohexyl)cyclohexyl)phenyl)ethenyl)-1-trifluoromethoxybenzene
Compound No.114
4'-(1-methyl-2-(4-(4-propylcyclohexyl)cyclohexyl)ethenyl)-3,4,5-trifluorobiphenyl
Compound No.115
4'-(1-methyl-2-(4-(4-propylcyclohexyl)cyclohexyl)ethenyl)-3,5-difluoro-4-trifluoromethylbiphenyl
Compound No.116
2',6'-difluoro-4'-(1-methyl-2-(4-(4-propylcyclohexyl)cyclohexyl) ethenyl)-3,5-difluoro-4-trifluoromethylbiphenyl
Compound No.117
4'-(1-methyl-4-(4-(4-propylcyclohexyl)cyclohexyl)-1-butenyl)-2-fluoro-1-trifluoromethoxybenzene
Compound No.118
4-(2-methyl-2-(4-(4-pentylcyclohexyl)phenyl)ethenyl)-1-trifluoro methoxybenzene
C 62.8 N 150.0 I

Example 3 (Use Example 1)

A liquid crystal composition B1 consisting of the following components was prepared.

| | |
|---|---|
| 4-(4-propylcyclohexyl)benzonitrile | 24% |
| 4-(4-pentylcyclohexyl)benzonitrile | 36% |
| 4-(4-heptylcyclohexyl)benzonitrile | 25% |
| 4-(4-(4-pentylcyclohexyl)phenyl)benzonitrile | 15% |

Clearing point of the nematic liquid crystal composition was 72.4 °C, threshold voltage at cell thickness of 9 microns was 1.78 V, dielectric anisotropy was 11.0, optical anisotropy was 0.137, and viscosity at 20°C was 27.0 mPaS. The liquid crystal composition (85%) was mixed with the compound of the present invention (Compound No. 64, 4-(1-methyl-2-(4-(4-propylcyclohexyl)cyclohexyl)ethenyl)-1,2-difluorobenzene) (15%) to prepare a liquid crystal composition A1.

Clearing point of the liquid crystal composition A1 was 74.9°C, threshold voltage at cell thickness of 8.7 microns was 1.68 V, dielectric anisotropy was 10.3, optical anisotropy was 0.131, viscosity at 20°C was 29.6 mPaS, and elastic coefficient ratio $K_{33}/K_{11}$ was 1.88.

The composition A1 was allowed to stand in a freezer at -20 °C for 60 days but crystal separation was not observed. Various values obtained by extrapolation were as follows. Clearing point : 89.1°C, dielectric anisotropy: 6.3, optical anisotropy: 0.098, viscosity: 45.0 mPaS. Voltage holding ratio of the composition A1 was 99.2% at 100°C.

Example 4 (Comparative Example 1)

Phase transition temperature of the compounds (Comparative Compounds 1 and 2) prepared according to the methods described in JP-B-3-22855 and JP-A-63-502756 was measured. The results are shown in Table 1 which shows that the compound of the present invention has more excellent liquid crystal characteristics and liquid crystal phase and higher clearing point than the compounds of the prior art.

Table 1

| | Phase transition temperature (°C) |
|---|---|
| 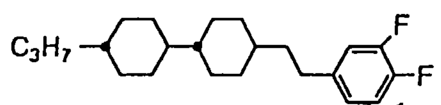 Comparative compound 1 | C 18.4 S 49.7 N 118 I |
| 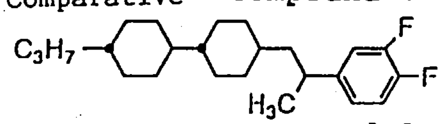 Comparative compound 2 | C r.t. or lower I |
| 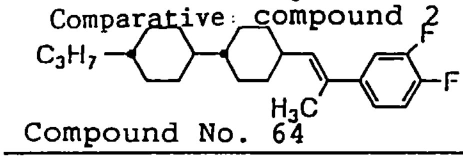 Compound No. 64 | C 56 N 120 I |

Example 5 (Comparative Example 2)

Various characteristics of the compound (Comparative Compound 3) prepared according to the method described in JP-B-3-22855 were measured under the same conditions as in Example 3 (Comparative Example 1). That is, dielectric anisotropy, optical anisotropy, elastic coefficient ratio $K_{33}/K_{11}$, and time elapsed before crystal separation when allowed in a freezer at -20°C were measured for Comparative Compound 3 and the compound of the present invention (Compound No.65). The results are shown in Table 2 .

Table 2

| | Dielectric anisotropy | Optical anisotropy | Elastic coefficient ratio | Time elapsed before crystal separation |
|---|---|---|---|---|
| 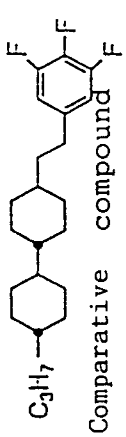 Comparative compound | 9.7 | 0.077 | 0.189 | longer than 40 days |
| 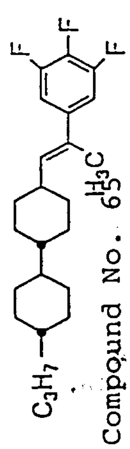 Compound No. 65 | 9.0 | 0.097 | 0.187 | 15 days |

Among the above values, those for dielectric anisotropy and optical anisotropy are obtained by extrapolation. Table 2 shows that the compound of the present invention has larger optical anisotropy and more excellent phase solubility than the compound of the prior art.

Example 6 (Use Example 2)

| | |
|---|---|
| 3-HHVMeB(F)-F | 9.0% |
| 3-HHVMeB(F,F)-F | 8.0% |
| 1V2-BEB(F,F)-C | 5.0% |
| 3-HB-C | 25.0% |
| 1-BTB-3 | 5.0% |
| 2-BTB-1 | 10.0% |
| 3-HH-4 | 11.0% |
| 3-HHB-1 | 11.0% |
| 3-H2BTB-2 | 4.0% |
| 3-HB(F)TB-2 | 6.0% |
| 3-HB(F)TB-3 | 6.0% |
| $T_{N1}$=81.4(°C)<br>η=19.8(mPa • s)<br>Δn=0.147<br>Δε=7.6<br>Vth=2.03(V) | |

Example 7 (Use Example 3)

| | |
|---|---|
| 3-BVMeB-4 | 10.0% |
| V2-HB-C | 12.0% |
| 1V2-HB-C | 12.0% |
| 3-HB-C | 14.0% |
| 3-HB(F)-C | 5.0% |
| 2-BTB-1 | 2.0% |
| 3-HH-4 | 8.0% |
| 3-HH-VFF | 6.0% |
| 2-HHB-C | 3.0% |
| 3-HHB-C | 6.0% |
| 3-HB(F)TB-2 | 8.0% |
| 3-H2BTB-2 | 5.0% |
| 3-H2BTB-3 | 5.0% |
| 3-H2BTB-4 | 4.0% |
| $T_{N1}$=81.6(°C)<br>η=15.8(mPa•s)<br>Δn=0.150<br>Δε=7.2<br>Vth=2.12(V) | |

Example 8 (Use Example 4)

| | |
|---|---|
| 3-BVMeB(F)-F | 8.0% |
| 2O1-BEB(F)-C | 5.0% |
| 3O1-BEB(F)-C | 15.0% |
| 4O1-BEB(F)-C | 13.0% |
| 5O1-BEB(F)-C | 13.0% |
| 2-HHB(F)-C | 15.0% |
| 3-HHB(F)-C | 15.0% |
| 3-HB(F)TB-4 | 4.0% |
| 3-HHB-1 | 8.0% |
| 3-HHB-O1 | 4.0% |
| $T_{N1}$=75.2(°C)<br>η=85.2(mPa•s)<br>Δn=0.137<br>Δε=30.7<br>Vth=0.84(V) | |

Example 9 (Use Example 5)

| | |
|---|---|
| 3-HHVMeB(F)-F | 5.0% |
| 3-HHVMeB(F,F)-F | 5.0% |
| 3-BVMeB-4 | 5.0% |
| 3-BVMeB(F)-F | 5.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 3-PyB-2 | 2.0% |
| 4-PyB-2 | 2.0% |
| 6-PyB-O5 | 3.0% |
| 6-PyB-O6 | 3.0% |
| 6-PyB-O7 | 3.0% |
| 6-PyB-O8 | 3.0% |
| 3-PyBB-F | 6.0% |
| 4-PyBB-F | 6.0% |
| 5-PyBB-F | 6.0% |
| 3-HHB-1 | 6.0% |
| 3-HHB-3 | 8.0% |
| 2-H2BTB-2 | 4.0% |
| 2-H2BTB-3 | 4.0% |
| $T_{N1}$=68.7(°C)<br>η=36.5(mPa • s)<br>Δn=0.170<br>Δε=6.6<br>Vth=2.09(V) | |

Example 10 (Use Example 6)

| | |
|---|---|
| 3-HHVMeB(F)-F | 10.0% |
| 3-HHVMeB(F,F)-F | 10.0% |
| 2-HHB(F)-F | 17.0% |
| 3-HHB(F)-F | 17.0% |
| 5-HHB(F)-F | 16.0% |
| 3-H2HB(F)-F | 5.0% |
| 2-HBB(F)-F | 6.0% |
| 3-HBB(F)-F | 6.0% |
| 5-HBB(F)-F | 13.0% |
| $T_{N1}$=97.4(°C)<br>η=29.4(mPa • s)<br>Δn=0.099<br>Δε=5.8<br>Vth=2.12(V) | |

Example 11 (Use Example 7)

| | |
|---|---|
| 3-HHVMeB(F,F)-F | 12.0% |
| 7-HB(F,F)-F | 4.0% |
| 4-H2HB(F,F)-F | 10.0% |
| 5-H2HB(F,F)-F | 10.0% |
| 3-HHB(F,F)-F | 10.0% |
| 4-HHB(F,F)-F | 5.0% |
| 3-HH2B(F,F)-F | 15.0% |
| 5-HH2B(F,F)-F | 10.0% |
| 3-HBB(F,F)-F | 12.0% |
| 5-HBB(F,F)-F | 12.0% |
| $T_{N1}$=74.4(°C)<br>η=29.8(mPa • s)<br>Δn=0.088<br>Δε=8.6<br>Vth=1.58(V) | |

Example 12 (Use Example 8)

| | |
|---|---|
| 3-BVMeB-4 | 4.0% |
| 3-BVMeB(F)-F | 4.0% |
| 3-HHVMeB(F)-F | 8.0% |
| 3-HHVMeB(F,F)-F | 8.0% |
| 3-HB-CL | 10.0% |
| 1O1-HH-5 | 5.0% |
| 5-HBB(F)-F | 14.0% |
| 4-HHB-CL | 8.0% |
| 5-HHB-CL | 8.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |
| 3-HB(F)VB-3 | 4.0% |
| $T_{N1}$=86.2(°C)<br>η=24.4(mPa • s)<br>Δn=0.123<br>Δε=5.1<br>Vth=2.28(V) | |

Example 13 (Use Example 9)

| | |
|---|---|
| 3-HHVMeB(F)-F | 5.0% |
| 3-HHVMeB(F,F)-F | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 11.0% |
| 4-HHB-OCF3 | 7.0% |
| 5-HHB-OCF3 | 5.0% |
| 3-HH2B-OCF3 | 4.0% |
| 5-HH2B-OCF3 | 4.0% |
| 3-HHB(F,F)-OCF3 | 5.0% |
| 5-HBB(F)-F | 10.0% |
| 3-HH2B(F)-F | 3.0% |
| 3-HB(F)BH-3 | 3.0% |
| 5-HBBH-3 | 3.0% |
| $T_{N1}$=84.2(°C)<br>η=16.7(mPa • s)<br>Δn=0.088<br>Δε=4.7<br>Vth=2.39(V) | |

In the above examples, notation of the compounds are according to the method described in Table 3.

Viscosity (η) was measured at 20.0°C, optical anisotropy (Δn), dielectric anisotropy (Δε) and threshold voltage (Vth) were measured at 25.0 °C.

The compounds of the formula (1) of the present invention:

1) show very large elastic coefficient ratio $K_{33}/K_{11}$,
2) show very high chemical stability,
3) are excellent in compatibility with other liquid crystal compounds, in particular, compatibility at low temperature, or
4) have large positive dielectric anisotropy, and
5) very high specific resistance (high voltage holding ratio) and good UV stability.

The compounds of the formula (1) of the present invention having such excellent characteristics can be used to obtain liquid crystal compositions having excellent characteristics.

Table 3 Notation of compounds

$$R-(A_1)-Z_1 \cdots\cdots Z_n-(A_n)-X$$

| 1) left terminal group R- | symbol |
|---|---|
| $C_nH_{2n+1}-$ | n— |
| $C_nH_{2n+1}O-$ | nO— |
| $C_nH_{2n+1}OC_mH_{2m}-$ | nOm— |
| $CH_2=CH-$ | V— |
| $CH_2=CHC_nH_{2n}-$ | Vn— |
| $C_nH_{2n+1}CH=CHC_mH_{2m}-$ | nVm— |
| $C_nH_{2n+1}CH=CHC_mH_{2m}CH=CHC_kH_{2k}-$ | nVmVk— |

| 3) linking group $-Z_1-$, $-Z_n-$ | symbol |
|---|---|
| $-C_2H_4-$ | 2 |
| $-C_4H_8-$ | 4 |
| $-COO-$ | E |
| $-C\equiv C-$ | T |
| $-CH=CH-$ | V |
| $-CF_2O-$ | CF2O |
| $-OCF_2-$ | OCF2 |
| $-CH=C(CH3)-$ | VMe |

| 2) ring structure $-(A_1)-$, $-(A_n)-$ | symbol |
|---|---|
| 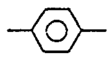 | B |
| 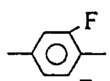 | B(F) |
| 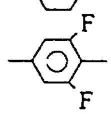 | B(F,F) |
| 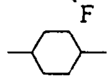 | H |
| 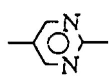 | Py |
| 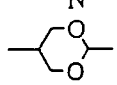 | D |
| 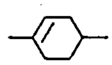 | Ch |

| 4) right terminal group -X | symbol |
|---|---|
| $-F$ | —F |
| $-Cl$ | —CL |
| $-CN$ | —C |
| $-CF_3$ | —CF3 |
| $-OCF_3$ | —OCF3 |
| $-OCF_2H$ | —OCF2H |
| $-C_nH_{2n+1}$ | —n |
| $-OC_nH_{2n+1}$ | —On |
| $-COOCH_3$ | —EMe |
| $-C_nH_{2n}CH=CH_2$ | —nV |
| $-C_mH_{2m}CH=CHC_nH_{2n+1}$ | —mVn |
| $-CH=CF_2$ | —VFF |
| $-CH=CHC_2H_4\text{-}F$ | —V2F |

5) notation example

3−H2B(F,F)B(F)−F

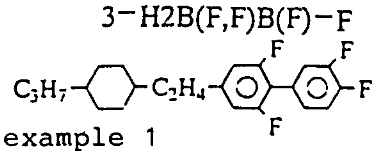

example 1

1V2−BEB(F,F)−C

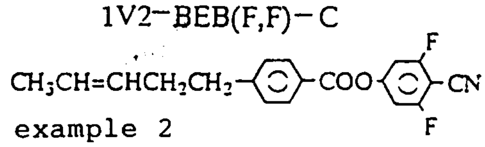

example 2

## Claims

1. A compound of the following formula (1):

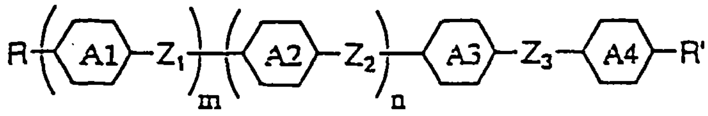 (1)

wherein R and R 'represent independently alkyl groups having 1 to 10 carbon atoms, cyano groups, halogen atoms, halogenated alkyl groups having 1 to 5 carbon atoms, or said alkyl groups or halogenated alkyl groups wherein at least one $-CH_2-$ is replaced by -O-, -S-, -CH=CH - or -C≡C-;
rings $A_1$, $A_2$, $A_3$ and $A_4$ represent independently benzene rings, cycloalkane rings having 3 to 8 carbon atoms, cycloalkene rings having 3 to 8 carbon atoms, bicycloalkane rings having 5 to 10 carbon atoms, benzene rings wherein at least one -CH= in the rings is replaced by -N=, -CF=, -CCl= or -CBr=, cycloalkane rings wherein at least one $-CH_2-$ in the rings is replaced by -O- or -S-, cycloalkene rings wherein at least one $-CH_2-$ in the rings is replaced by -O- or -S- and/or at least one - CH= in the rings is replaced by -N=, or bicycloalkane rings wherein at least one $-CH_2-$ in the rings is replaced by -O- or -S- and/or at least one -CH= in the rings is replaced by -N=;
at least one of $Z_1$, $Z_2$ and $Z_3$ represents an alkenylene group of the following formula Q:

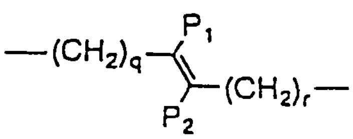 Q

wherein $P_1$ and $P_2$ represent independently hydrogen atoms or alkyl groups having 1 to 10 carbon atoms, provided that at least one of $P_1$ and $P_2$ represent alkyl groups having 1 to 10 carbon atoms, q and r represent independently 0, 1 or 2 and the sum of q + r is 2 or less; and the other of $Z_1$, $Z_2$ and $Z_3$ represent independently a covalent bond, $-CH_2CH_2-$, - CH=CH -, -C≡C-, -COO-, -OCO-, $-CH_2O-$, $-OCH_2-$, $-CF_2O-$, $-OCF_2-$, $-(CH_2)_4-$, $-CH=CH-(CH_2)_2-$, $-CH_2CH=CHCH_2-$ or $-(CH_2)_2-CH=CH-$;
m and n represent independently 0 or 1, provided that if m is 0, at least one of $Z_2$ and $Z_3$ is Q and if both m and n are 0, $Z_3$ is Q.

2. The compound of claim 1 wherein one of $P_1$ and $P_2$ is methyl group and the other is hydrogen atom.

3. The compound of claim 1 wherein both q and r are 0.

4. A liquid crystal composition which comprises at least one of the compounds as defined in any one of claims 1 to 3.

5. A liquid crystal composition which comprises at least one of the compounds as defined in any one of claims 1 to 3 as a first component, and at least one compound selected from the group consisting of the compounds of following formulas (2), (3) and (4) as a second component:

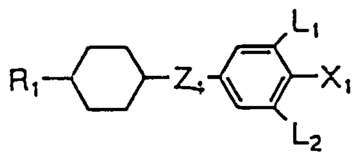

(2)

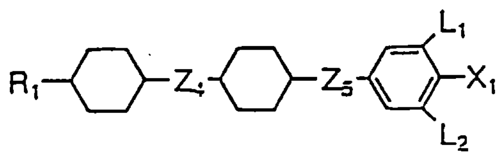

(3)

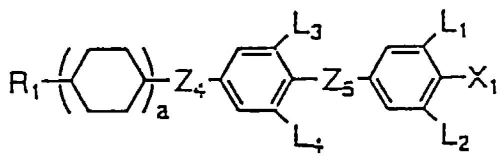

(4)

wherein $R_1$ represents an alkyl group having 1 to 10 carbon atoms, $X_1$ represents F, Cl, $OCF_3$, $OCF_2H$, $CF_3$, $CF_2H$ or $CFH_2$, $L_1$, $L_2$, $L_3$ and $L_4$ represent independently H or F, $Z_4$ and $Z_5$ represent independently - $(CH_2)_2$-, -CH=CH- or covalent bonds, and a is 1 or 2.

6. A liquid crystal composition which comprises at least one of the compounds as defined in any one of claims 1 to 3 as a first component and at least one compound selected from the group consisting of the compounds of following formulas (5), (6), (7), (8) and (9) as a second component:

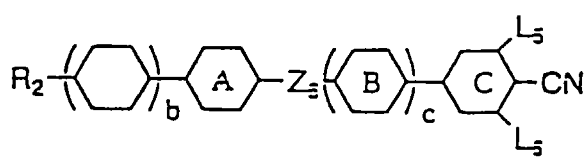

(5)

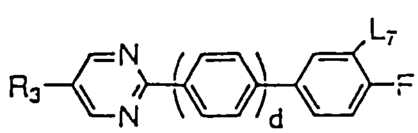

(6)

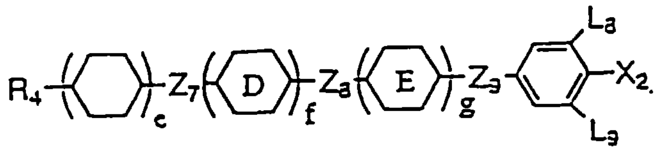

(7)

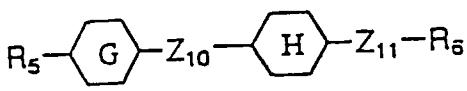

(8)

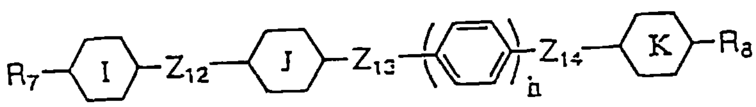

(9)

wherein, in the formula (5), $R_2$ is F or an alkyl group having 1 to 10 carbon atoms or an alkenyl group having 2 to 10 carbon atoms, wherein any methylene group ($-CH_2-$) in said alkyl or alkenyl group may be replaced by oxygen atom (-O-), provided that two or more successive methylene groups are not replaced by oxygen atoms, ring A represents trans-1,4-cyclohexylene, 1,4-phenylene or 1,3-dioxane-2,5-diyl group, ring B represents trans-1,4-cyclohexylene, 1,4-phenylene or pyrimidine-2,5-diyl group, ring C represents trans-1,4-cyclohexylene, or 1,4-phenylene group, $Z_6$ represents $-(CH_2)_2-$, -COO- or a covalent bond, $L_5$ and $L_6$ represent independently H or F, and b and c represent independently 0 or 1;

in the formula (6), $R_3$ represents an alkyl group having 1 to 10 carbon atoms, $L_7$ represents H or F, and d is 0 or 1;

in the formula (7), $R_4$ represents an alkyl group having 1 to 10 carbon atoms, rings D and E represent independently trans-1,4-cyclohexylene or 1,4-phenyelne group, $Z_7$ and $Z_8$ represent independently -COO- or covalent bonds, $Z_9$ represents -COO- or -C≡C-, $L_8$ and $L_9$ represent independently H or F, $X_2$ represents F, $OCF_3$, $OCF_2H$, $CF_3$, $CF_2H$ or $CFH_2$, provided that if $X_2$ is $OCF_3$, $OCF_2H$, $CF_3$, $CF_2H$ or $CFH_2$, both $L_8$ and $L_9$ are H, e, f and g represent independently 0 or 1;

in the formula (8), $R_5$ and $R_6$ represent independently alkyl groups having 1 to 10 carbon atoms or alkenyl groups having 2 to 10 carbon atoms, any methylene group ($-CH_2-$) in said alkyl or alkenyl group may be replaced by oxygen atom (-O-), provided that two or more successive methylene groups are not replaced by oxygen atoms, ring G represents trans-1,4-cyclohexylene, 1,4-phenylene or pyrimidine-2,5-diyl group, ring H represents trans-1,4-cyclohexylene or 1,4-phenylene group, $Z_{10}$ represents -C≡C-, -COO-, $-(CH_2)_2-$, -CH=CH-C≡C- or a covalent bond, $Z_{11}$ represents -COO- or a covalent bond;

in the formula (9), $R_7$ and $R_8$ represent independently alkyl groups having 1 to 10 carbon atoms or alkenyl groups having 2 to 10 carbon atoms, any methylene group ($-CH_2-$) in said alkyl or alkenyl group may be replaced by oxygen atom (-O-), provided that two or more successive methylene groups are not replaced by oxygen atoms, ring I represents. trans-1,4-cyclohexylene, 1,4-phenylene or pyrimidine-2,5-diyl group, ring J represents trans-1,4-cyclohexylene group, 1,4-phenylene group wherein one or more hydrogen atoms on the ring may be replaced by F, or pyrimidine-2,5-diyl group wherein one or more hydrogen atoms on the ring may be replaced by F, ring K represents trans-1,4-cyclohexylene or 1,4-phenylene group, $Z_{12}$ and $Z_{14}$ represent independently -COO-, $-(CH_2)_2-$or covalent bonds, $Z_{13}$ represents -CH=CH-, -C≡C-, -COO- or a covalent bond, and h is 0 or 1.

**7.** A liquid crystal composition which comprises at least one of the compounds as defined in any one of claims 1 to 3 as a first component, at least one compound selected from the group consisting of the compounds of the formulas (2), (3) and (4) as a second component and at least one compound selected from the group consisting of the compounds of the formulas (5), (6), (7), (8) and (9) as another of the second component.

**8.** A liquid crystal display element comprising the liquid crystal composition as defined in any one of claims 4 to 7.

**INTERNATIONAL SEARCH REPORT**

International application No.
PCT/JP96/01626

**A. CLASSIFICATION OF SUBJECT MATTER**

Int. Cl$^6$ C07C13/28, C07C15/50, C07C15/52, C07C22/04, C07C25/24, C07C43/192, C07C43/225, C07C43/188, C07C43/215,

According to International Patent Classification (IPC) or to both national classification and IPC

**B. FIELDS SEARCHED**

Minimum documentation searched (classification system followed by classification symbols)

Int. Cl$^6$ C07C13/28, C07C15/50, C07C15/52, C07C22/04, C07C25/24, C07C43/192, C07C43/225, C07C43/188, C07C43/215,

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X<br>Y | JP, 47-13216, A (International Business Machines Corp.), July 5, 1972 (05. 07. 72) & DE, 2165700, A & FR, 2120740, A & GB, 1324468, A & US, 3767289, A | 1-4, 8<br>5 - 7 |
| Y | JP, 4-330019, A (F. Hoffmann - La Roche AG.), November 18, 1992 (18. 11. 92), Claim; page 14, right column, line 23 to page 18, right column, line 4 & EP, 442306, A2 & US, 5204018, A | 1 - 8 |
| X | Chem. Abstr., Vol. 77, No. 1, 3 July 1972 (Columbus, OH, USA), page 85-86, the abstract No. 14579x, OWEN, NORRIS V. 'Papillomatous growths on internal genitalia···' Toxicol. Appl. Pharmacol. 1972, 21(4), 582-5(Eng). | 1 - 2 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| September 10, 1996 (10. 09. 96) | September 17, 1996 (17. 09. 96) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP96/01626

A. (Continuation) CLASSIFICATION OF SUBJECT MATTER

C07C69/757, C07C69/75, C07C255/50, C07C255/54, C09K19/16, C09K19/30, C09K19/42, G02F1/13

B. (Continuation) FIELDS SEARCHED

C07C69/757, C07C69/75, C07C255/50, C07C255/54, C09K19/16, C09K19/30, C09K19/42, G02F1/13

Form PCT/ISA/210 (extra sheet) (July 1992)